# EUROPEAN PATENT SPECIFICATION

(11) **EP 1 472 347 B1**
(45) Date of publication and mention of the grant of the patent: **12.01.2011**
(21) Application number: 03707645.2
(22) Date of filing: 31.01.2003
(51) Int. Cl.: C12N 15/00, C12N 15/09, C12Q 1/68, C07H 21/04

(54) **IMPROVED PROTEIN EXPRESSION IN BACILLUS SUBTILIS**
VERBESSERTE PROTEINEXPRESSION IN BACILLUS SUBTILIS
ACCROISSEMENT DE L EXPRESSION DE PROTEINES DANS LE BACILLUS SUBTILIS

(30) Priority: 15.02.2002 US 357558 P
(43) Date of publication of application: 03.11.2004
(73) Proprietor: Danisco US Inc., Palo Alto, CA 94304 (US)
(72) Inventor: BRON, Sierd, NL-9752 JL Haren (NL); FERRARI, Eugenio, San Bruno, CA 94066 (US); TJALSMA, Harold, NL-6561 CH Groesbeek (NL)
(74) Representative: Kiddle, Simon John
(86) International application number: PCT/US2003/002961
(87) International publication number: WO 2003/070963

(56) References cited:
- US-A- 5 387 521
- US-B1- 6 509 185
- PEREGO M ET AL: "Pentapeptide regulation of aspartyl-phosphate phosphatases" PEPTIDES 2001 UNITED STATES, vol. 22, no. 10, 2001, pages 1541-1547, XP002367983 ISSN: 0196-9781
- KOETJE EMMO J ET AL: "A plasmid-borne Rap-Phr system of Bacillus subtilis can mediate cell-density controlled production of extracellular proteases." MICROBIOLOGY (READING), vol. 149, no. 1, January 2003 (2003-01), pages 19-28, XP002367984 ISSN: 1350-0872
- POTTATHIL MRIDULA ET AL: "The extracellular Phr peptide-Rap phosphatase signaling circuit of Bacillus subtilis." FRONTIERS IN BIOSCIENCE, vol. 8, 1 January 2003 (2003-01-01), pages d32-45, XP002367985 ISSN: 1093-4715
- TJALSMA H ET AL: "Engineering of quorum-sensing systems for improved production of alkaline protease by Bacillus subtilis." JOURNAL OF APPLIED MICROBIOLOGY, vol. 96, no. 3, 2004, pages 569-578, XP002367986 ISSN: 1364-5072
- MUELLER J.P. ET AL: 'TRANSCRIPTIONAL REGULATION OF BACILLUS-SUBTILIS GLUCOSE STARVATION-INDUCIBLE GENES CONTROL OF GSIA BY THE COMP-COMA SIGNAL TRANSDUCTION SYSTEM' JOURNAL OF BACTERIOLOGY vol. 174, no. 13, July 1992, pages 4361 - 4373
- JIANG M. ET AL: 'Differential processing of propeptide inhibitors of Rap phosphatases in Bacillus subtilis' JOURNAL OF BACTERIOLOGY vol. 182, no. 2, January 2000, pages 303 - 310

## Description

### FIELD OF THE INVENTION

The present invention relates to cells that have been genetically manipulated to have an altered capacity to produce expressed proteins. In particular, the present invention relates to Gram-positive microorganisms having exogenous nucleic acid sequences introduced therein and methods for producing proteins in such host cells, such as members of the genus *Bacillus.* More specifically, the present invention relates to the expression, production and secretion of a polypeptide of interest and to cells that have been genetically manipulated to have an altered capacity to produce expressed proteins. In particular, the present invention provides for the enhanced expression of a selected polypeptide by a microorganism.

### BACKGROUND OF THE INVENTION

Gram-positive microorganisms, such as members of the genus *Bacillus,* have been used for large-scale industrial fermentation due, in part, to their ability to secrete their fermentation products into their culture media. Secreted proteins are exported across a cell membrane and a cell wall, and then are subsequently released into the external media.

Indeed, secretion of heterologous polypeptides is a widely used technique in industry. Typically, cells are transformed with a nucleic acid encoding a heterologous polypeptide of interest to be expressed and thereby produce large quantities of desired polypeptides. This technique can be used to produce a vast amount of polypeptide over what would be produced naturally. These expressed polypeptides have a number of industrial applications, including therapeutic and agricultural uses, as well as use in foods, cosmetics, cleaning compositions, animal feed, etc. Thus, increasing expression of polypeptides is of great interest in many fields.

The Gram-positive eubacterium *Bacillus subtilis* has acquired the ability to survive in a complex and continuously changing environment. To address such changes in its environment, *B. subtilis* has developed complex signal transduction systems to sense a wide variety of extracellular stimuli. During its life cycle, *B. subtilis* uses two-component regulatory systems, each consisting of a sensor kinase and a response regulator (mostly a transcription (actor) for processes such as competence development Qubnan, Microbiol, Rev., 55:395-424 [1991]), synthesis of peptide antibiotics (Marahiel et al., Mol. Microbiol., 7:631-636 [1993]), production of secreted proteolytic enzymes (Kunst et al., Res. Microbiol., 145: 393-402 [1994]), and sporulation (Grossman, Ann. Rev. Genet., 29:477-508 [1995]). Upon phosphorylation of a response regulator by its cognate sensor kinase, transcription of a specific subset of genes is induced (Fabret et al., J. Bacteriol., 181:1975-1983 [1999]; and Jiang et al., Mol. Microbiol., 38:535-542 [2000]). Fine-tuning of these regulatory systems can be governed by intracellular response regulators aspartyl ***p***hosphatases (Raps), and their antagonistic ***ph***osphatase regulators (Phrs) that serve as cell-density signalling molecules (Perego et al., Mol. Microbiol., 19:1151-1170 [1996]; and Perego, Cell 76:1047-1055 [1998]). The Rap phosphatases have the ability to dephosphorylate their cognate response regulators, thereby temporally "overruling" the action of sensor kinases (Gray, Trends Microbiol., 5:184-188 [1997]).

Most *B. subtilis* 168 *rap* genes are transcribed in operons with genes that encode their antagonistic Phr peptides (Kunst et al., Nature 390:249-56 [1997]). While the Rap phosphatases remain in the cytoplasm, Phr peptides contain an amino-terminal signal peptide and have the potential to be exported as pro-peptides, most likely *via* the Sec pathway (Perego *et al.,* [1996] *supra*; Tjalsma et al., Mol. Biol. Rev., 64:515-547 [2000]). Further possible extracellular processing may result in active Phr pentapeptides with a weakly conserved X**R**XX**T** sequence. These pentapeptides are thought to be re-imported *via* the ***o***ligo***p***eptide ***p***ermease (Opp) system, and specifically inhibit the activity of their cognate Rap phosphatase (Solomon et al., Genes Dev., 10:2014-2024 [1996]; Perego, Proc. Natl. Acad. Sci. USA 94:8612-8617 [1997]; and Perego [1998] *supra).* However, four of the eleven known *rap* genes are not followed by functional *phr* genes, suggesting that these may not be involved in quorum-sensing processes. Interestingly, *rap-phr* operons were also found on several endogenous rolling-circle plasmids from *B. subtilis* (Meijer et al., FEMS Microbiol. Lett., 37:283-288 [1998]).

The chromosomally-encoded Rap-Phr systems orchestrate the sequential programmes of global gene transcription in a growing culture of *B. subtilis* cells. During exponential growth (low cell-densities), RapC dephosphorylates ComA-P, thereby inhibiting competence development. At the end of the exponential growth phase, the activity of RapC is antagonized by the accumulation of the PhrC peptide, and competence development is stimulated (Lazazzera et al., Cell, 89: 917-925 [1997]; and Lazazzera et al., J. Bacteriol., 181: 5193-5200 [1999]). Under these conditions, sporulation is repressed, due to the stimulation of *rapA* and *rapE* expression via ComA∼P. This results in high cellular levels of RapA and RapE that together dephosphorylate SpoOF (Perego *et al*., [1996] *supra*; and Jiang et al., J. Bacteriol., 182: 303-310 [2000]). During the post-exponential growth phase, when nutrients become limiting, RapA and RapE are antagonised by PhrA and PhrE, respectively, and sporulation is initiated *via* SpoOF∼P. Although the production of degradive enzymes is known to be increased during high cell-density growth *via* the DegS-DegU two-component system, no involvement of chromosomally-encoded Rap phosphatases in the repression of protein secretion at low cell-densities has previously been documented. Recently, the pTA1060-encoded phosphatase Rap60 was shown to repress the secretion of proteolytic enzymes in *B. subtilis* 168 (Koetje et al., Microbiology, 149: 19-28 [2003]). Expression of *aprE,* encoding the major extracellular proteolytic enzyme subtilisin, was strongly repressed in cells containing Rap60. However, co-production of Phr60, or the addition of synthetic Phr60 peptide, restored *aprE* transcription under these conditions (Koetje *et al*., *supra*). The regulatory cascade affected by Rap60 comprises, most likely, components of the phosphorelay (Hoch, J. Cell Biochem., 51:55-61 [1993]) and the transition-state regulator AbrB (Strauch and Hoch, Mol. Microbiol., 7:337-342 [1993]). Thus, plasmids endogenous to *B. subtilis* contain Rap-Phr systems that have a function in the cell-density controlled production of, at least one, expressed protease. However, the role of the chromosomal Rap-Phr system in protein expression remains unknown.

### SUMMARY OF THE INVENTION

The present invention relates to cells that have been genetically manipulated to have an altered capacity to produce expressed proteins. In particular, the present invention relates to Gram-positive microorganisms having exogenous nucleic acid sequences introduced therein and methods for producing proteins in such host cells, such as members of the genus *Bacillus.* More specifically, the present invention relates to the expression, production and secretion of a polypeptide of interest and to cells that have been genetically manipulated to have an altered capacity to produce expressed proteins. In particular, the present invention provides for the enhanced expression of a selected polypeptide by a microorganism.

In some embodiments, the present invention provides means for increasing the transcription, production and/or secretion of a protein in a cell comprising proteins involved in the Rap-Phr systems.

In one embodiment, the present invention provides means for the hyperexpression (*i*.*e*., overexpression) of one or more of the *phr* genes, except *phrC,* which leads to enhanced expression of a polypeptide of interest.

In a further embodiment of the invention, enhanced expression of a polypeptide of interest from a host cell is induced by contacting the host cell with a composition comprising one or more Phr proteins or pentapeptides. The Phr proteins or pentapeptides derived therefrom are selected from the group consisting of PhrA, PhrE, PhrF, PhrI, and PhrK.

In additional embodiments, the present invention provides means for the inactivation of one or more of the *rap* genes or gene products, except *rapC,* which leads to enhanced expression of a polypeptide of interest. In some embodiments, the gene is inactivated by homologous recombination, while in other embodiments, inactivation is accomplished via *in vivo* mutagenesis, *in vitro* mutagenesis, or any other method known in the art. In some preferred embodiments a *rap* gene product is mRNA, while in other embodiments, it is the protein product. In some embodiments, the mRNA is inactivated by contacting with an antisense RNA, an RNAse or an RNA binding protein that blocks translation. In some embodiments, a Rap protein is non-functional due to a modification in the rap gene, the presence of an antagonist and/or a blocker molecule. Any method that prevents the functional expression of one or more of the *rap* genes is contemplated herein.

In one embodiment, the polypeptide of interest is a homologous/endogenous polypeptide that is native or naturally occurring in the host cell, the transcription/expression of which is controlled, directly or indirectly, by the *spoA* gene product.

In another embodiment, the polypeptide of interest is a heterologous/exogenous polypeptide that is not naturally occurring in the host cell.

Further disclosed herein are host cells wherein one or more of the *rap* genes, except *rapC,* have a decreased level of transcription and/or translation of the *rap* gene.

### DESCRIPTION OF THE FIGURES

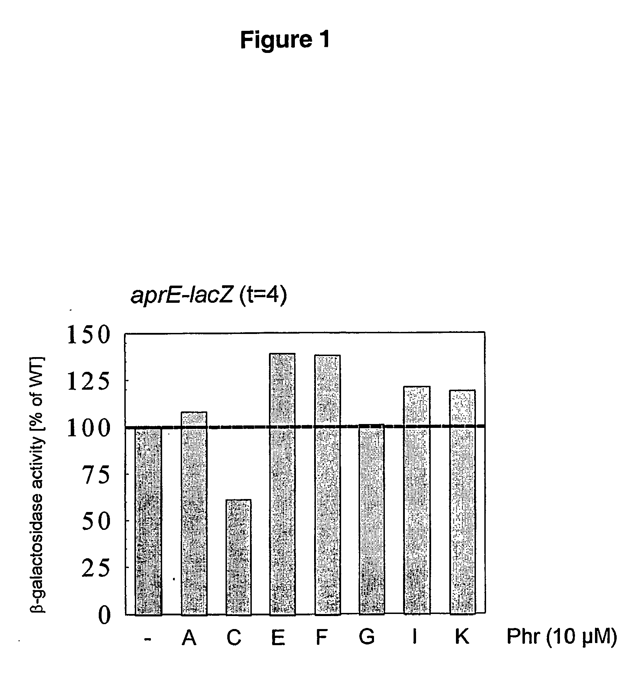

Figure 1 provides a bar graph depicting the different effects of Phr pentapeptides on *aprE* transcription. The transcription of the *aprE-IacZ* gene fusion in *B. subtilis* AL grown in TY medium at 37°C in the presence of 10 µM of one of the following pentapeptides (see Table II): PhrA (A), PhrC, (C), PhrE (E), PhrF (F), PhrG (G), PhrI (I) or PhrK (K), or in the absence of pentapeptides (-), was measured 4 hours after the transition point between the exponential and post-exponential growth phases. β-galactosidase activities were determined in Units per OD₆₀₀ (% of WT; *aprE-lacZ* transcription level of the strain grown in the absence of Phr peptides [WT] is set to 100%).

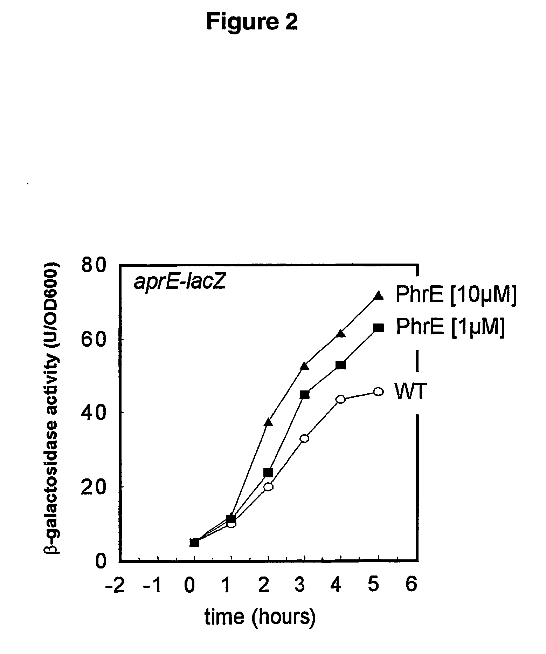

Figure 2 provides a graph showing that PhrE stimulates *aprE* transcription. Time courses of the transcription of the *aprE-lacZ* gene fusion in *B. subtilis* AL grown in TY medium with at 37°C, without PhrE peptide (O; WT), and with 1 µM PhrE (►), or 10 µM PhrE (■) peptide added to the medium were determined. β-galactosidase activities were determined in Units per OD₆₀₀. Zero time (T₀) indicates the transition point between the exponential and post-exponential growth phases.

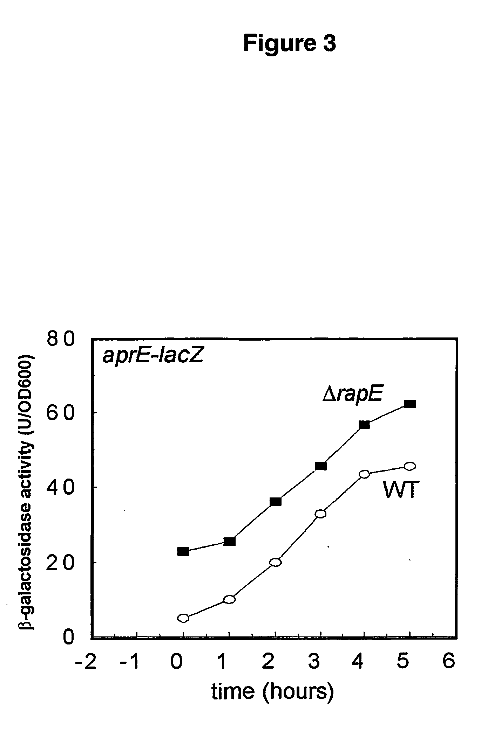

Figure 3 shows that RapE is a repressor of *aprE* transcription. Time courses of the transcription of the *aprE-lacZ* gene fusion in *B. subtilis* AL (○; WT), or *B. subtilis ΔrapE* AL (■; *ΔrapE*) grown in TY medium at 37°C were determined. β-galactosidase activities were determined in Units per OD₆₀₀. Zero time (T₀) indicates the transition point between the exponential and post-exponential growth phases.

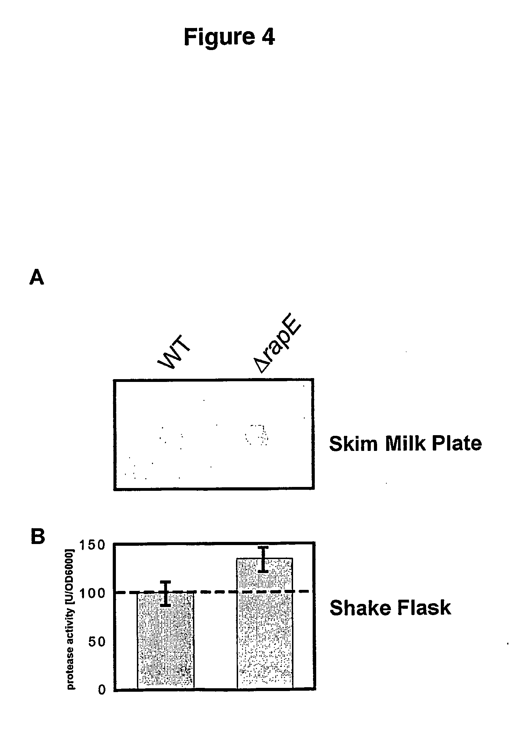

Figure 4 shows that there is improved protease secretion in a *rapE* mutant strain. Panel A shows results of monitoring extracellular protease production on plates. In these experiments, *B. subtilis* 168 *degU32(Hy)* (WT) and *B. subtilis 168 ΔrapE degU32(Hy) (ΔapE)* were grown overnight at 37°C on TY-agar plates containing 1% skim milk. The size of the halo's around the colonies reflects the level of proteolytic activity secreted into the medium. Panel B shows results of monitoring extracellular protease activity in shake flask cultures. In these experiments, *B. subtilis* 168 *degU32*(Hy) (wild-type), and *B. subtilis* 168 *ΔrapE degU32*(Hy) (*ΔrapE*) were grown overnight at 37°C in TY-medium. Next, proteolytic activity in the medium was determined using azo dye impregnated collagen (see: experimental procedures section; values are the average of three individual experiments).

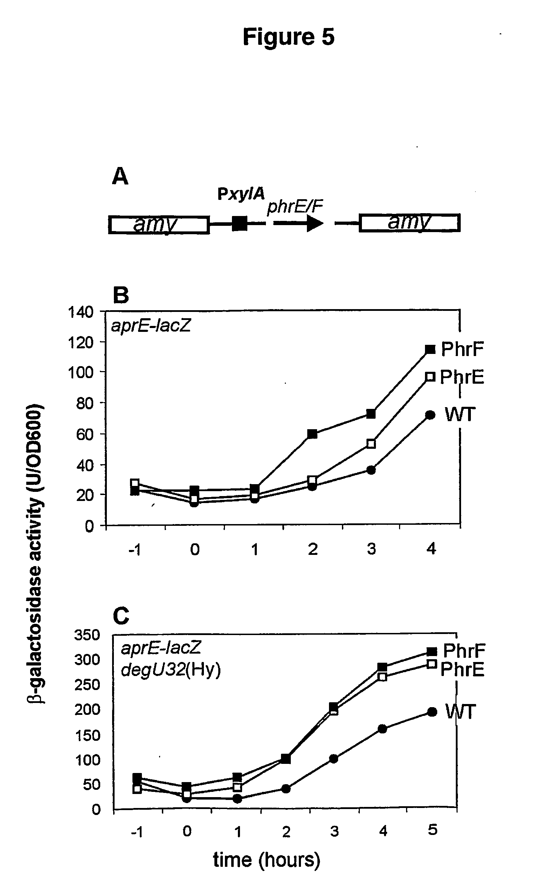

Figure 5, Panel A shows a schematic representation of the chromosome of *B. subtilis XphrE* and *XphrF,* in which the *phrE* or *phrf* genes are integrated in the *amyE* gene and are under control of the xylose-inducible *Pxyl* promoter.

Figure 5, Panel B and Panel C show that over-production of PhrE and PhrF can increase *aprE* transcription. Time courses of the transcription of the *aprE-lacZ* gene fusion in *B. subtilis* AL (•; WT), *B. subtilis XphrE* AL (□; PhrE) or *B. subtilis XphrF* AL (■; PhrF) and in, (B) *B. subtilis* AL *degU32*(Hy) (•; WT), *B. subtilis XphrE* AL *degU32*(Hy) (□; PhrE), or *B. subtilis XphrF* AL *degU32*(Hy) (■; PhrF), grown in TY medium at 37°C were determined. β-gatactosidase activities were determined in Units per OD₆₀₀. Zero time (T₀) indicates the transition point between the exponential and post-exponential growth phases.

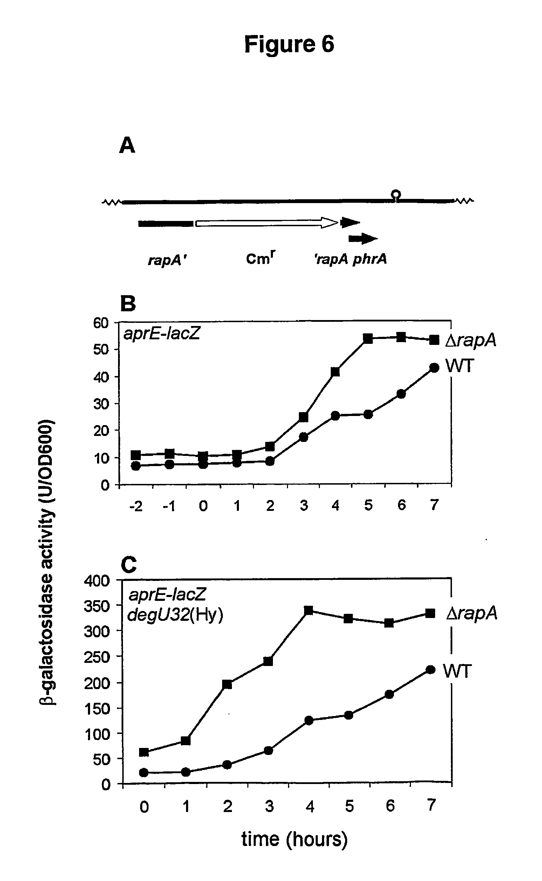

Figure 6, Panels A, B and C provide information regarding rapA. Panel A shows a schematic representation of the chromosome of *B. subtilis ΔrapA,* in which the *rapA* gene is disrupted with a chloramphenicol marker. Figure 6, Panel B and Panel C show that RapA is a repressor of *aprE* transcription. Time courses of the transcription of the *aprE-lacZ* gene fusion in *B. subtilis* AL (•; WT), or *B. subtilis ΔrapA* AL (■; *ΔrapA*), and *B. subtilis* AL *degU32(Hy)* (•; WT), or *B. subtilis ΔrapA* AL *degU32*(Hy) (■; *ΔrapA*) (Panel B) grown in TY medium at 37°C were determined. β-galactosidase activities were determined in Units per OD₆₀₀. Zero time (T₀) indicates the transition point between the exponential and post-exponential growth phases.

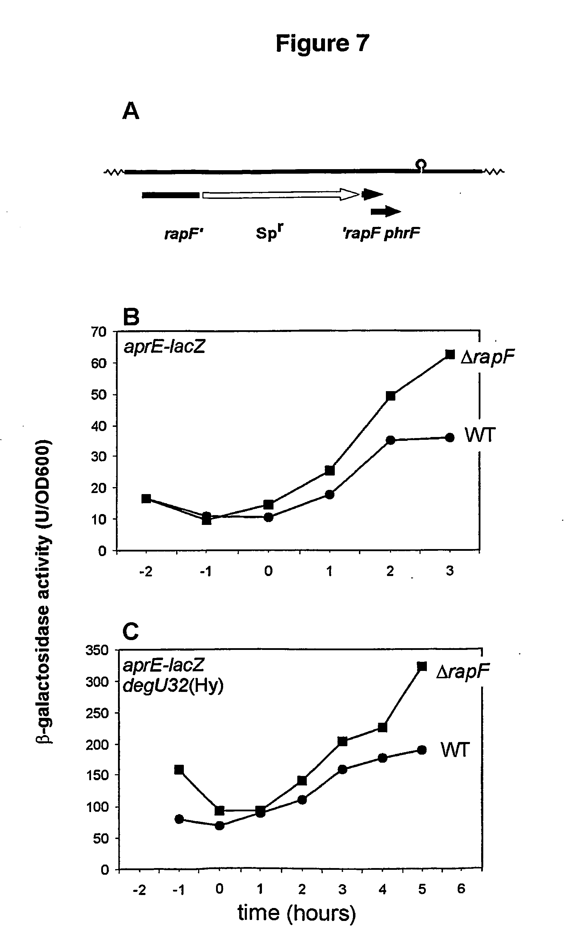

Figure 7, Panel A shows a schematic representation of the chromosome of *B. subtilis ΔrapF,* in which the *rapF* gene is disrupted with a spectinomycin marker. Figure 7, Panel B and Panel C show that RapF is a repressor of *aprE* transcription. Time courses of the transcription of the *aprE-lacZ* gene fusion in *B. subtilis* AL (•; WT), or *B. subtilis ΔtapF* AL (■; *ΔrapF*) and in Panel B, *B. subtilis* AL *degU32*(Hy) (•; WT), or *B. subtilis ΔrapF* AL *degU32*(Hy) (■; *ΔrapF*) grown in TY medium at 37°C were determined. β-galactosidase activities were determined in Units per OD₆₀₀. Zero time (T₀) indicates the transition point between the exponential and post-exponential growth phases.

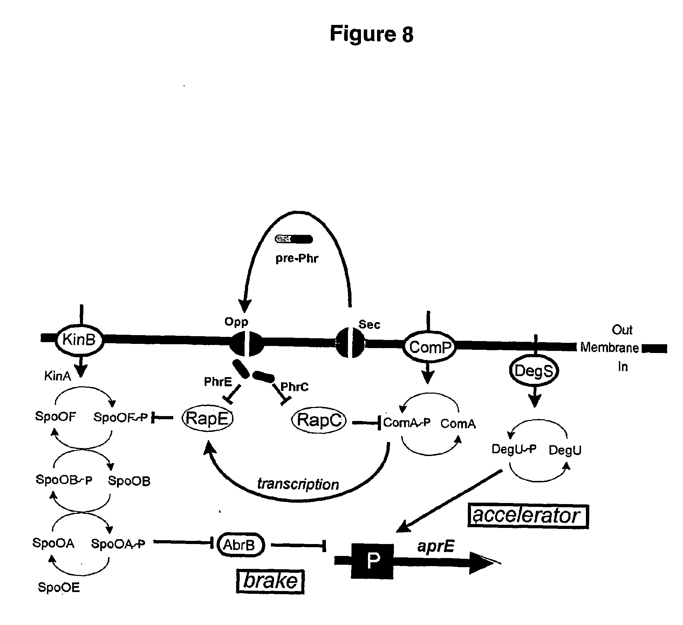

Figure 8 depicts a theoretical model for Rap-Phr-mediated regulation of AprE transcription. RapE is able to dephosphorylate SpoOF∼P, components of the phosphorelay (SpoOF-SpoOB-SpoOA), thereby lowering the cellular levels of SpoOA∼P. This results in low levels of *aprE* transcription, possibly *via* AbrB (Koetje *et al*., *supra*). At high cell-densities, sufficient PhrE has been accumulated in the medium to be taken up in sufficient amounts by the Opp system. This results in RapE inhibition, more phosphorylated SpoOA and, eventually, increased *aprE* transcription. Notably, the addition of extracellular PhrC inhibits *aprE* transcription, possibly due to higher cellular levels of ComA-P causing a higher transcription level of *rapE.* Thus, Rap-Phr systems function as a break on the transcription of the *aprE* gene during the early growth phases (exponential growth) of the cell. After entry into the post-exponential growth phase this brake is, at least partly, removed by the accumulation of certain Phr peptides, and *aprE* transcription is further stimulated by DegU∼P (accelerator).

Figure 9 shows representative structures of transforming DNA, as described herein. In the top panel, homology boxes are shown flanking an incoming sequence. In the middle panel, other non-homologous sequences are shown to be added to the ends. In the bottom panel, the ends are closed such that the transforming DNA forms a closed circle or loop.
The bottom representation also encompasses the transforming DNA construct incorporated into a vector.

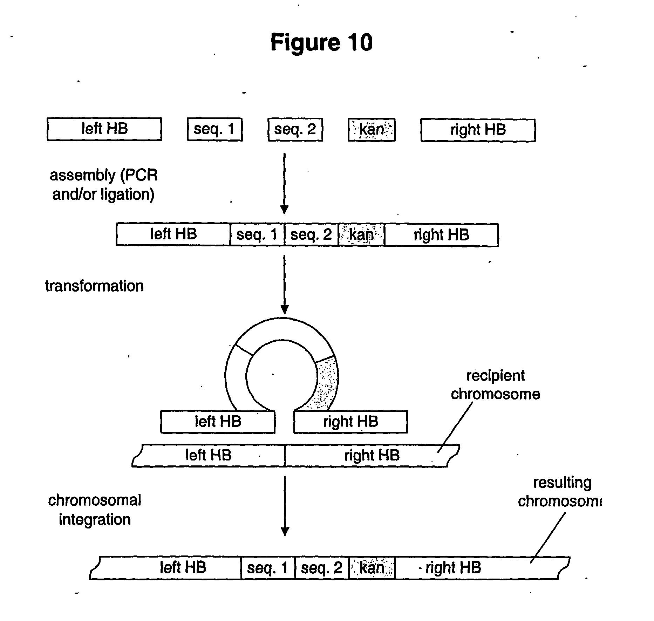

Figure 10 provides a schematic diagram showing cloning by *in vitro* assembly and transformation of a competent *Bacillus,* in accordance with the present invention. For example, in some embodiments sequence 1 ("seq 1") is a *phr* gene, and sequence 2 ("seq 2") is a gene encoding a polypeptide of interest. Although the kanamycin (kan) gene is depicted as the selection marker in this Figure, it is contemplated that any suitable selection maker finds use in the present invention.

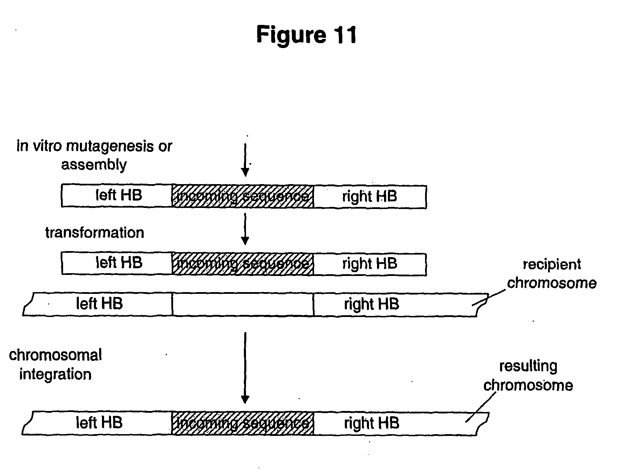

Figure 11 is a schematic diagram illustrating the insertion of a mutagenized incoming sequence into the host cell chromosome by homologous recombination. It should be noted that maximizing the homology between the transforming DNA and the target region of the chromosome can increase the transformation efficiency. In some preferred embodiments, the mutagenized sequence encodes a non-functional *rap* gene, one or more copies of the *phr* gene, a polypeptide of interest DNA sequence, etc.

Figure 12 shows the DNA sequence for *rapA.*

Figure 13 shows the deduced amino acid for RapA.

Figure 14 shows the DNA sequence for *phrA*.

Figure 15 shows the deduced amino acid for PhrA.

Figure 16 shows the DNA sequence for *rapB* .

Figure 17 shows the deduced amino acid for RapB.

Figure 18 shows the DNA sequence for *rapC.*

Figure 19 shows the deduced amino acid for RapC.

Figure 20 shows the DNA sequence for *phrC.*

Figure 21 shows the deduced amino acid for PhrC.

Figure 22 shows the DNA sequence for *rapE*.

Figure 23 shows the deduced amino acid for RapE.

Figure 24 shows the DNA sequence for *phrE*.

Figure 25 shows the deduced amino acid for PhrE.

Figure 26 shows the DNA sequence for *rapF.*

Figure 27 shows the deduced amino acid for RapF .

Figure 28 shows the DNA sequence for *phrF.*

Figure 29 shows the deduced amino acid for PhrF .

Figure 30 shows the DNA sequence for *rapG .*

Figure 31 shows the deduced amino acid for RapG.

Figure 32 shows the DNA sequence for *phrG .*

Figure 33 shows the deduced amino acid for PhrG.

Figure 34 shows the DNA sequence for *rapl*.

Figure 35 shows the deduced amino acid for Rapl .

Figure 36 shows the DNA sequence for *phrl*.

Figure 37 shows the deduced amino acid for Phrl.

Figure 38 shows the DNA sequence for *rapK*.

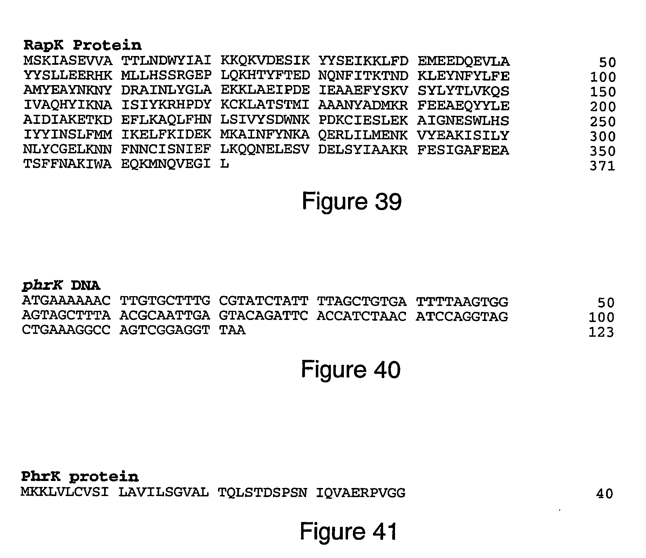

Figure 39 shows the deduced amino acid for RapK.

Figure 40 shows the DNA sequence for *phrK.*

Figure 41 shows the deduced amino acid for PhrK.

### DESCRIPTION OF THE INVENTION

The present invention relates to cells that have been genetically manipulated to have an altered capacity to produce expressed proteins. In particular, the present invention relates to Gram-posltive microorganisms having exogenous nucleic acid sequences Introduced therein and methods for producing proteins in such host cells, such as members of the genus *Baclllus.* More specifically, the present invention relates to the expression, production and secretion of a polypeptide of Interest and to cells that have been genetically manipulated to have an altered capacity to produce expressed proteins. In particular, the present Invention provides for the enhanced expression of a selected polypeptide by a microorganism.

Unless defined otherwise herein, all technical and scientific terms used herein have the same meaning as commonly understood by one of ordinary skill in the art to which this invention belongs (*See e.g*., Singleton, et al., DICTIONARY OF MICROBIOLOGY AND MOLECULAR BIOLOGY, 2D ED., John Wiley and Sons, New York [1994]; and Hale & Marham, THE HARPER COLLINS DICTIONARY OF BIOLOGY, Harper Perennial, NY [1991], both of which provide one of skill with a general dictionary of many of the terms used herein). Although any methods and materials similar or equivalent to those described herein can be used in the practice or testing of the present invention, the preferred methods and materials are described. Numeric ranges are inclusive of the numbers defining the range. Unless otherwise indicated, nucleic acids are written left to right in 5' to 3' orientation; amino acid sequences are written left to right in amino to . ' carboxy orientation, respectively. The headings provided herein are not limitations of the various aspects or embodiments of the invention that can be had by reference to the specification as a whole. Accordingly, the terms defined immediately below are more fully defined by reference to the specification as a whole.

### Definitions

As used herein, "host cell" refers to a cell that has the capacity to act as a host and expression vehicle for an expression cassette as described herein. In one embodiment, the host cell is a Gram positive microorganism. In some preferred embodiments, the term refers to cells in the genus *Bacillus.* As used herein, the genus *Bacillus* includes all members known to those of skill in the art, including but not limited to *B. subtilis, B. licheniformis, B. lentus, B. brevis, B. stearothermophilus, B. alkalophilus, B. amyloliquefaciens, B. clausii, B. halodurans, B. megaterum, B. coagulans, B. ciculans, B. lautus,* and *B. thuringiensis.*

The term "polypeptide," as used herein, refers to a compound made up of amino acid residues linked by peptide bonds. The term "protein" as used herein, may be synonymous with the term "polypeptide" or may refer, in addition, to a complex of two or more polypeptides. Thus, the terms "protein," "peptide," and "polypeptide" are used interchangeably.

Additionally, a "protein of interest," or "polypeptide of interest," refers to the protein/polypeptide to be expressed and secreted by the host cell. The protein of interest may be any protein that up until now has been considered for expression in prokaryotes and/or eukaryotes. In one embodiment, the protein of interest which is translocated by the secretion-associated proteins or systems utilized by the host cell include proteins comprising a signal peptide. The protein of interest may be either homologous or heterologous to the host. In some embodiments, the protein of interest is a secreted polypeptide, particularly an enzyme which is selected from amylolytic enzymes, proteolytic enzymes, cellulytic enzymes, oxidoreductase enzymes and plant wall degrading enzymes. In further embodiments, these enzyme include amylases, proteases, xylanases, lipases, laccases, phenol oxidases, oxidases, cutinases, cellulases, hemicellulases, esterases, perioxidases, catalases, glucose oxidases, phytases, pectinases, glucosidases, isomerases, transferases, galactosidases and chitinases. In still further embodiments, the expressed polypeptide is a hormone, growth factor, receptor, vaccine, antibody, or the like. While it is not intended that the present invention be limited to any particular protein/polypeptide, in some most preferred embodiments, the expressed polypeptide is a protease.

As used herein, the terms "chimeric polypeptide" and "fusion polypeptide" are used interchangeably to refer to a protein that comprises at least two separate and distinct regions that may or may not originate from the same protein. For example, a signal peptide linked to the protein of interest wherein the signal peptide is not normally associated with the protein of interest would be termed a chimeric polypeptide or chimeric protein.

As used herein, the terms "chimeric DNA construct" and "heterologous nucleic acid construct," refer to a gene (*i*.*e*., one that has been introduced into a host) that is comprised of parts of different genes, including regulatory elements. Thus, in some embodiments, a chimeric gene is an endogenous gene operably linked to a promoter that is not its native promoter. A chimeric gene construct for transformation of a host cell is typically composed of a transcriptional regulatory region (promoter) operably linked to a protein coding sequence, or, in a selectable marker chimeric gene, to a selectable marker gene encoding a protein conferring antimicrobial resistance to transformed cells. In one embodiment, a typical chimeric gene construct which is useful for transformation into a host cell comprises a transcriptional regulatory region that is constitutive or inducible, a signal peptide coding sequence, a protein coding sequence, and a terminator sequence. In other embodiments, the chimeric gene comprises a promoter operably linked to a *phr* gene. In yet other embodiments, the chimeric gene comprises a promoter operably linked to a gene encoding a protein of interest. In still further embodiments, chimeric gene constructs also comprise a second DNA sequence encoding a signal peptide if secretion of the target protein is desired.

A "signal peptide," as used herein, refers to an amino-terminal extension on a protein to be secreted. "Signal sequence" is used interchangeably herein. In most preferred embodiments, secreted proteins use an amino-terminal protein extension which plays a crucial role in the targeting to and translocation of precursor proteins across the membrane and which is proteolytically removed by a signal peptidase during or immediately following membrane transfer.

As used herein, the term "enhanced" refers to improved production of proteins of interest. In preferred embodiments, the present invention provides enhanced (*i*.*e*., improved) production and secretion of a protein of interest. In these embodiments, the "enhanced" production is improved as compared to the normal levels of production by the host (*e.g*., wild-type cells). Thus, for heterologous proteins, basically any expression is enhanced, as the cells normally do not produce the protein.

As used herein, the terms "isolated" and "purified" refer to a nucleic acid or amino acid that is removed from at least one component with which it is naturally associated.

As used herein, the term "heterologous protein" refers to a protein or polypeptide that does not naturally occur in a host cell. Examples of heterologous proteins include enzymes such as hydrolases including proteases, cellulases, amylases, other carbohydrases, and lipases; isomerases such as racemases, epimerases, tautomerases, or mutases; transferases, kinases and phosphatases, hormones, growth factors, cytokines, antibodies and the like. Thus, in some embodiments, heterologous proteins comprise therapeutically significant protein or peptides (*e.g*., growth factors, cytokines, ligands, receptors and inhibitors), as well as vaccines and antibodies. In alternate embodiments, the protein is a commercially important industrial protein or peptide. It is intended that the term encompass protein that are encoded by naturally occurring genes, mutated genes, and/or synthetic genes.

As used herein, the terms "heterologous nucleic acid construct" and "heterologous nucleic acid sequence" refer to a portion of a genetic sequence that is not native to the cell in which it is expressed. "Heterologous," with respect to a control sequence refers to a control sequence (*i*.*e*., promoter or enhancer) that does not function in nature to regulate the same gene the expression of which it is currently regulating. Generally, heterologous nucleic acid sequences are not endogenous to the cell or part of the genome in which they are present, and have been added to the cell, by infection, transfection, microinjection, electroporation, or the like. In some embodiments, "heterologous nucleic acid constructs" contain a control sequence/DNA coding sequence combination that is the same as, or different from a control sequence/DNA coding sequence combination found in the native cell.

As used herein, the term "homologous protein" refers to a protein or polypeptide native or naturally occurring in a host cell. The present disclosure encompasses host cells producing the homologous protein via recombinant DNA technology. The present disclosure further encompasses a host cells with one or more deletions or one or more interruptions of the nucleic acid encoding the naturally occurring homologous protein or proteins, such as, for example, a protease, and having nucleic acid encoding the homologous protein or proteins re-introduced in a recombinant form (*i*.*e*., in an expression cassette). In other embodiments, the host cell produces the homologous protein.

As used herein, the term "nucleic acid molecule" includes RNA, DNA and cDNA molecules. It will be understood that, as a result of the degeneracy of the genetic code, a multitude of nucleotide sequences encoding a given protein may be produced.

As used herein, the term "vector" refers to a nucleic acid construct designed for transfer between different host cells. An "expression vector" refers to a vector that has the ability to incorporate and express heterologous DNA fragments in a foreign cell. Many prokaryotic and eukaryotic expression vectors are commercially available. Selection of appropriate expression vectors is within the knowledge of those having skill in the art.

As used herein, the terms "expression cassette" and "expression vector" refer to a nucleic acid construct generated recombinantly or synthetically, with a series of specified nucleic acid elements that permit transcription of a particular nucleic acid in a target cell. The recombinant expression cassette can be incorporated into a plasmid, chromosome, mitochondrial DNA, plastid DNA, virus, or nucleic acid fragment. Typically, the recombinant expression cassette portion of an expression vector includes, among other sequences, a nucleic acid sequence to be transcribed and a promoter.

As used herein, the term "plasmid" refers to a circular double-stranded (ds) DNA construct used as a cloning vector, and which forms an extrachromosomal self-replicating genetic element in many bacteria and some eukaryotes.

As used herein, the term "selectable marker-encoding nucleotide sequence" refers to a nucleotide sequence which is capable of expression in the host cells and where expression of the selectable marker confers to cells containing the expressed gene the ability to grow in the presence of a corresponding selective agent or lack of an essential nutrient.

As used herein, the term "selectable marker" refers to a gene capable of expression in host cell which allows for ease of selection of those hosts containing the vector. Examples of such selectable markers include but are not limited to antimicrobials, (*e.g*., kanamycin, erythromycin, actinomycin, chloramphenicol and tetracycline). Thus, the term "selectable marker" refers to genes that provide an indication that a host cell has taken up an incoming DNA of interest or some other reaction has occurred. Typically, selectable markers are genes that confer antimicrobial resistance or a metabolic advantage on the host cell to allow cells containing the exogenous DNA to be distinguished from cells that have not received any exogenous sequence during the transformation. A "residing selectable marker" is one that is located on the chromosome of the microorganism to be transformed. A residing selectable marker encodes a gene that is different from the selectable marker on the transforming DNA construct.

As used herein, the term "promoter" refers to a nucleic acid sequence that functions to direct transcription of a downstream gene. In preferred embodiments, the promoter is appropriate to the host cell in which the target gene is being expressed. The promoter, together with other transcriptional and translational regulatory nucleic acid sequences (also termed "control sequences") is necessary to express a given gene. In general, the transcriptional and translational regulatory sequences include, but are not limited to, promoter sequences, ribosomal binding sites, transcriptional start and stop sequences, translational start and stop sequences, and enhancer or activator sequences.

A nucleic acid is "operably linked" when it is placed into a functional relationship with another nucleic acid sequence. For example, DNA encoding a secretory leader (*i*.*e*., a signal peptide), is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

As used herein, the term "gene" means the segment of DNA involved in producing a polypeptide chain, that may or may not include regions preceding and following the coding region (*e.g*. 5' untranslated (5' UTR) or "leader" sequences and 3' UTR or "trailer" sequences), as well as intervening sequences (introns) between individual coding segments (exons).

In some embodiments, the gene encodes therapeutically significant proteins or peptides, such as growth factors, cytokines, ligands, receptors and inhibitors, as well as vaccines and antibodies. The gene may encode commercially important industrial proteins or peptides, such as enzymes (*e.g*., proteases, carbohydrases such as amylases and glucoamylases, cellulases, oxidases and lipases). However, it is not intended that the present invention be limited to any particular enzyme or protein. In some embodiments, the gene of interest is a naturally-occurring gene, a mutated gene or a synthetic gene.

A nucleic acid sequence is considered to be "selectively hybridizable" to a reference nucleic acid sequence if the two sequences specifically hybridize to one another under moderate to high stringency hybridization and wash conditions. Hybridization conditions are based on the melting temperature I of the nucleic acid binding complex or probe. For example, "maximum stringency" typically occurs at about Tm-5°C (5° below the Tm of the probe); "high stringency" at about 5-10°C below the Tm; "intermediate stringency" at about 10-20°C below the Tm of the probe; and "low stringency" at about 20-25°C below the Tm. Functionally, maximum stringency conditions may be used to identify sequences having strict identity or near-strict identity with the hybridization probe; while high stringency conditions are used to identify sequences having about 80% or more sequence identity with the probe.

Moderate and high stringency hybridization conditions are well known in the art. An example of high stringency conditions includes hybridization at about 42°C in 50% formamide, 5X SSC, 5X Denhardt's solution, 0.5% SDS and 100 µg/ml denatured carrier DNA followed by washing two times in 2X SSC and 0.5% SDS at room temperature and two additional times in 0.1X SSC and 0.5% SDS at 42°C.

As used herein, "recombinant" includes reference to a cell or vector, that has been modified by the introduction of a heterologous nucleic acid sequence or that the cell is derived from a cell so modified. Thus, for example, recombinant cells express genes that are not found in identical form within the native (non-recombinant) form of the cell or express native genes that are otherwise abnormally expressed, under expressed or not expressed at all as a result of deliberate human intervention. "Recombination, "recombining," or generating a "recombined" nucleic acid is generally the assembly of two or more nucleic acid fragments wherein the assembly gives rise to a chimeric gene.

As used herein, the terms "transformed," "stably transformed," and "transgenic" used in reference to a cell means the cell has a non-native (heterologous) nucleic acid sequence integrated into its genome or as an episomal plasmid that is maintained through two or more generations.

As used herein, the term "expression" refers to the process by which a polypeptide is produced based on the nucleic acid sequence of a gene. The process includes both transcription and translation.

As used herein, the term "introduced" used in the context of inserting a nucleic acid sequence into a cell, means "transfection," "transformation," or "transduction," and includes reference to the incorporation of a nucleic acid sequence into a eukaryotic or prokaryotic cell where the nucleic acid sequence may be incorporated into the genome of the cell (*e.g*., chromosome, plasmid, plastid, or mitochondrial DNA), converted into an autonomous replicon, or transiently expressed (for example, transfected mRNA).

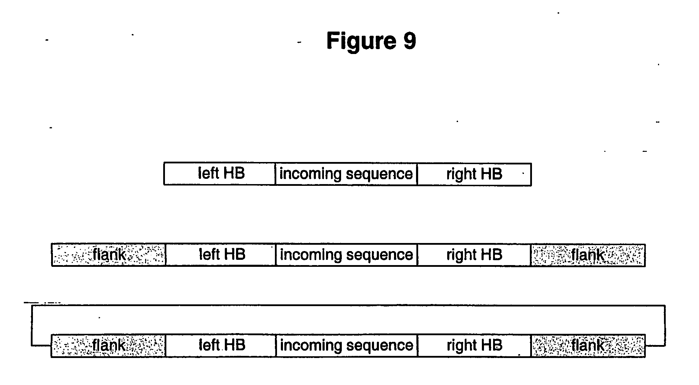

As used herein, "transforming DNA," "transforming sequence," and "DNA construct" refer to DNA that is used to introduce sequences into a host cell or organism. Transforming DNA is DNA used to introduce sequences into a host cell or organism. The DNA may be generated *in vitro* by PCR or any other suitable techniques. A typical structure of transforming DNA is shown in a schematic form in Figure 9. The transforming DNA comprises an incoming sequence. It may further comprise an incoming sequence flanked by homology boxes. In a further embodiment, the transforming DNA may comprise other non-homologous sequences, added to the ends (*i*.*e*., stuffer sequences or flanks). The ends can be closed such that the transforming DNA forms a closed circle, such as, for example, insertion into a vector.

In a preferred embodiment, mutant DNA sequences are generated with site saturation mutagenesis in at least one codon. In another preferred embodiment, site saturation mutagenesis is performed for two or more codons. In a further embodiment, mutant DNA sequences have more than 40%, more than 45%, more than 50%, more than 55%, more than 60%, more than 65%, more than 70%, more than 75%, more than 80%, more than 85%, more than 90%, more than 95%, or more than 98% homology with the wild-type sequence. In alternative embodiments, mutant DNA is generated *in vivo* using any known mutagenic procedure such as, for example, radiation, nitrosoguanidine and the like. The desired DNA sequence is then isolated and used in the methods provided herein.

In an alternative embodiment, the transforming DNA sequence comprises homology boxes without the presence of an incoming sequence. In this embodiment, it is desired to delete the endogenous DNA sequence between the two homology boxes. Furthermore, in some embodiments, the transforming sequences are wild-type, while in other embodiments, they are mutant or modified sequences. In addition, in some embodiments, the transforming sequences are homologous, while in other embodiments, they are heterologous.

As used herein, the term "incoming sequence" refers to a DNA sequence that is introduced into the *Bacillus* chromosome or genome. In preferred embodiments, the incoming sequence encodes one or more proteins of interest. In some embodiments, the incoming sequence comprises a sequence that may or may not already be present in the genome of the cell to be transformed (*i*.*e*., it may be either a homologous or heterologous sequence). In some embodiments, the incoming sequence encodes one or more proteins of interest, a gene, and/or a mutated or modified gene. In some embodiments, the incoming sequence includes a selectable marker, such as a gene that confers resistance to an antimicrobial.

In one embodiment, the incoming sequence encodes at least one heterologous protein including, but not limited to hormones, enzymes, and growth factors. In another embodiment, the enzyme includes, but is not limited to hydrolases, such as protease, esterase, lipase, phenol oxidase, permease, amylase, pullulanase, cellulase, glucose isomerase, laccase and protein disulfide isomerase.

In alternative embodiments, the incoming sequence encodes a functional wild-type gene or operon, a functional mutant gene or operon, or a non-functional gene or operon. In some embodiments, the non-functional sequence may be inserted into a gene to disrupt function of the gene.

As used herein, the term "target sequence" refers to a DNA sequence in the host cell that encodes the sequence where it is desired for the incoming sequence to be inserted into the host cell genome. In some embodiments, the target sequence encodes a functional wild-type gene or operon, while in other embodiments the target sequence encodes a functional mutant gene or operon, or a non-functional gene or operon.

As used herein, a "flanking sequence" refers to any sequence that is either upstream or downstream of the sequence being discussed (*e.g*., for genes A B C, gene B is flanked by the A and C gene sequences). In a preferred embodiment, the incoming sequence is flanked by a homology box on each side. In another embodiment, the incoming sequence and the homology boxes comprise a unit that is flanked by stuffer sequence on each side. In some embodiments, a flanking sequence is present on only a single side (either 3' or 5'), but in preferred embodiments, it is on each side of the sequence being flanked. The sequence of each homology box is homologous to a sequence in the *Bacillus* chromosome. These sequences direct where in the *Bacillus* chromosome the new construct gets integrated and what part of the *Bacillus* chromosome will be replaced by the incoming sequence.

As used herein, the term "stuffer sequence" refers to any extra DNA that flanks homology boxes (typically vector sequences). However, the term encompasses any non-homologous DNA sequence. Not to be limited by any theory, a stuffer sequence provides a noncritical target for a cell to initiate DNA uptake.

As used herein, the term "chromosomal integration" refers to the process whereby the incoming sequence is introduced into the chromosome of a host cell (*e.g., Bacillus).* The homology boxes of the transforming DNA align with homologous regions of the chromosome. Subsequently, the sequence between the homology boxes is replaced by the incoming sequence in a double crossover (*i*.*e*., homologous recombination).

As used herein, the term "homologous recombination" refers to the exchange of DNA fragments between two DNA molecules or paired chromosomes (*e.g*., during crossing over) at the site of identical nucleotide sequences. In a preferred embodiment, chromosomal integration is by homologous recombination.

As used herein, the term "library of mutants" refers to a population of cells which are identical in most of their genome but include different homologues of one or more genes. Such libraries can be used, for example, to identify genes or operons with improved traits.

As used herein, the terms "hypercompetent" and "super competent" mean that greater than 1 % of a cell population is transformable with chromosomal DNA (*e.g., Bacillus* DNA). Alternatively, the terms are used in reference to cell populations in which greater than10% of a cell population is transformable with a self-replicating plasmid (*e.g.,* a *Bacillus* plasmid). Preferably, the super competent cells are transformed at a rate greater than observed for the wild-type or parental cell population. Super competent and hypercompetent are used interchangeably herein.

The present disclosure provides means for the expression, production and secretion of polypeptides of interest, as well as cells that have been genetically manipulated to have an altered capacity to produce expressed proteins. In particular, the involvement of the Rap-Phr system in protein expression and secretion is described herein. As indicated, in preferred embodiments of the present invention, enhancement in expression and/or secretion of a polypeptide of interest is mediated by chromosomally-encoded Rap-Phr systems. In one embodiment, the polypeptide of interest is a secretory protease. In a specific embodiment the RapE-PhrE system encoded by the *B. subtilis* 168 chromosome is involved in the control of transcription of the *aprE* gene. In other embodiments, the extracellular addition of a synthetic Phr pentapeptide increases protein expression. In yet another embodiment, the disruption of a *rap* gene mediates a significant increase in protein expression. In one preferred embodiment, the protein is AprE. In a further embodiment, the expression and/or secretion of proteolytic enzymes is significantly increased in a strain carrying a *degU32*(Hy) mutation, in which the *rapE* gene is disrupted.

Many industrially important products (*e.g*., enzymes, hormones, growth factors, and other proteins) are produced from members of the genus *Bacillus* in large scale fermentation processes, including without limitation, proteases, lipases, amylases, and beta-glucanases. These products (*i*.*e*., proteins of interest) are either homologous or heterologous to the host. For homologous proteins, "overexpression" refers to expression above normal levels in the host cell. For heterologous proteins, any expression is of course "overexpression." Thus, it is advantageous to have a cell that will fail to sporulate yet possesses enhanced expression of gene(s) of interest.

*Bacillus subtilis* uses two-component signal transduction systems to sense intra-, and extracellular stimuli to adapt to environmental changes. Regulator aspartate phosphatases (Rap) have important roles in these processes, as they can dephosphorylate certain response-regulators, and are themselves subject to cell-density controlled inhibition by secreted Phr peptides. In *B. subtilis,* this family of Rap phosphatases is encoded by eleven chromosomal genes. During the development of the present invention it was determined that the addition of certain synthetic Phr pentapeptides, among which PhrE, to the growth medium of *B. subtilis* 168 improved the transcription of the *aprE* gene, encoding a major extracellular protease. Similarly, disruption of the *B. subtilis* 168 *rapE* gene improved the transcription of the *aprE* gene. Furthermore, the expression and/or secretion of proteolytic enzymes was shown to be significantly increased in a strain in which the *rapE* mutation was combined with a *degU32* (Hy) mutation for hyper secretion. Thus, Rap-Phr quorum-sensing mechanisms enable *B. subtilis* to suppress protease secretion under conditions of low-cell densities, as according in their natural habitat. However, under (highcell density) fermentation conditions, Rap phosphatases, such as RapE, might actually repress protease production. Thus, the modulation of cellular levels of certain Rap phosphatases finds use in improving the yields of *Bacillus* production strains. However, it is not intended that the present invention be limited to any particular mechanism(s).

Data obtained during the development of the present invention show that at least some chromosomally-encoded Rap-Phr systems are involved in the regulation of certain genes and protein expression and/or secretion. Although the experiments were focused on the RapE-PhrE system, it is contemplated that other chromosomally-encoded Rap-Phr systems are also involved in cell-density controlled protein expression and/or secretion. Thus, it is not intended that the present invention be limited to any particular mechanisms. However, this hypothesis is corroborated by some lines of evidence. First, as RapE is known to be involved in the cell-density controlled dephosphorylation of SpoOF∼P (Jiang et al., J. Bacteriol., 182:303-310 [2000]), other Rap phosphatases that are known to be acting on SpoOF∼P, such as RapA and RapB (Perego, [1998], *supra*; and Tzeng et al., Biochem., 37:16538-16545 [1998]), are likely candidates to be involved in *aprE* transcription. Second, experiments concerning the extracellular addition of synthetic Phr peptides indicated that at least one other Rap phosphatase of unknown function, RapF, has an effect on *aprE* transcription similar to that of RapE (*See*, Figure 1). Third, experiments to assess the extracellular addition of Phr peptides indicated that RapC antagonizes the function of RapE. As RapC is known to be involved in the transcription of the *rapA* and *rapE* genes, *via* the dephosphorylation of ComA-P (Jiang *et al*., [2000], *supra*), this is most likely an indirect effect of higher cellular levels of RapA and RapE. Figure 8 provides a putative model for Rap-Phr-mediated *aprE* transcription. However, as indicated throughout the present Specification, it is not intended that the present invention be limited to any particular mechanism(s). RapE is able to dephosphorylate SpoOF∼P, a component of the phosphorelay (SpoOF-SpoOB-SpoOA), thereby lowering the cellular levels of SpoOA∼P. At high cell-densities, sufficient PhrE has been accumulated in the medium to be taken up in sufficient amounts. This results in RapE inhibition, more phosphorylated SpoOA and, eventually, increased *aprE* transcription. Notably, extracellularly added PhrC inhibits *aprE* transcription, possibly due to higher cellular levels of ComA-P causing a higher transcription level of *rapE.* Thus, Rap-Phr systems function as a brake on the transcription of the *aprE* gene during the early growth phases (exponential growth) of the cell. After entry into the post-exponential growth phase this brake is, at least partly, removed by the accumulation of certain Phr peptides, and *aprE* transcription is further stimulated by DegU∼P.

Together, these results indicate that Rap-Phr systems of *B. subtilis* are suitable for exploitation in cell-density controlled production of industrial proteins. The potential power of these systems is that they function as a brake on the transcription of genes, such as *aprE,* during the early growth phases (exponential growth) of the cell. By modulating the cellular levels of Rap/Phr, this brake time can, conceivably, be shortened or extended. The latter is desirable for the production of deleterious proteins which inhibit the growth of *B. subtilis.* By delaying the initiation of transcription of the genes for such proteins until the (late) post-exponential growth phase, the growth inhibitory effect is be of minor importance. In contrast, the removal of certain Rap-Phr systems, such as RapE-PhrE, removes part of the brake during the exponential growth phases, which is contemplated to have an positive effect on bulk enzyme production. Notably, the present results indicate that overproduction of RapC might have a similar effect on protease secretion as the depletion of RapE.

### B. subtilis rap and phr Nucleotide Sequences

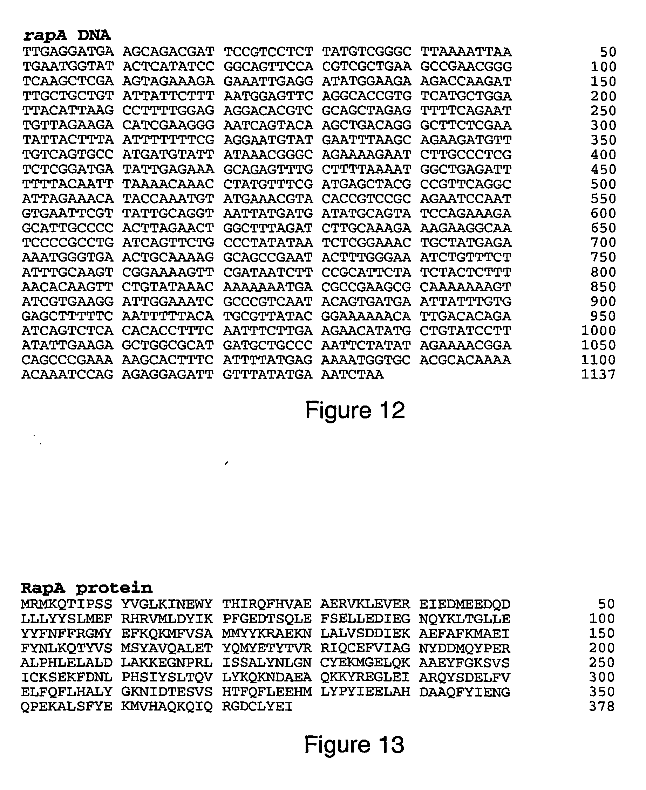

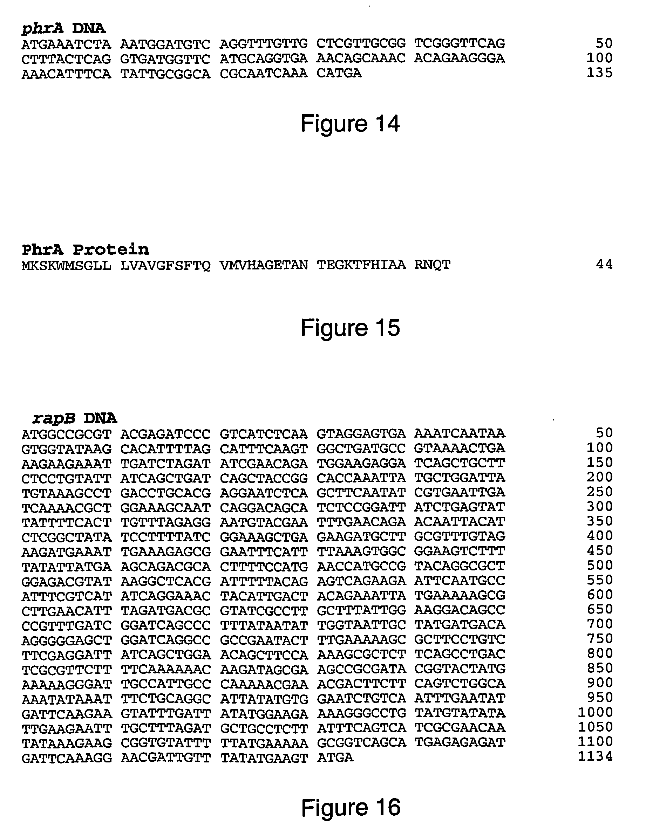

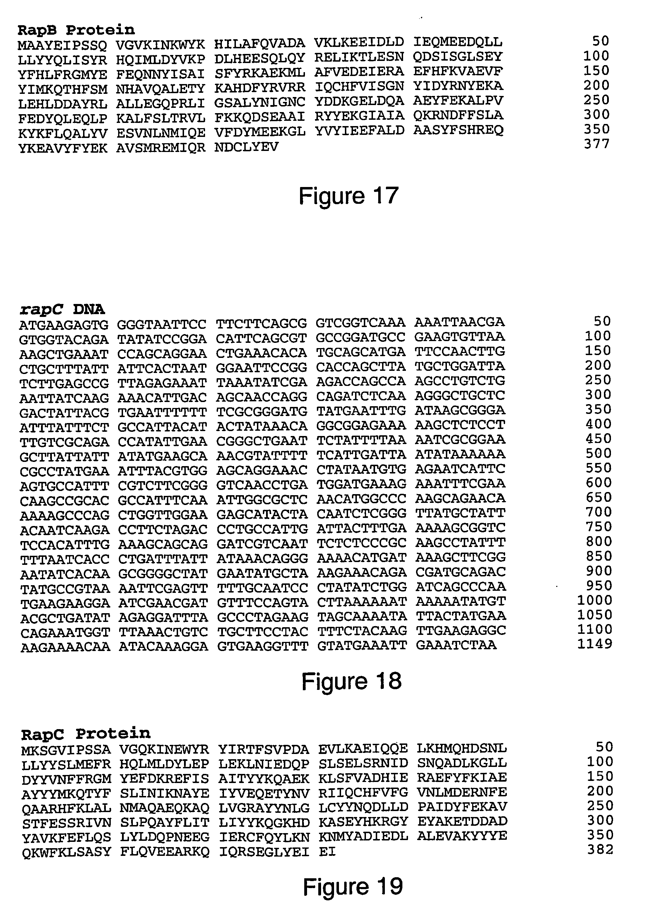

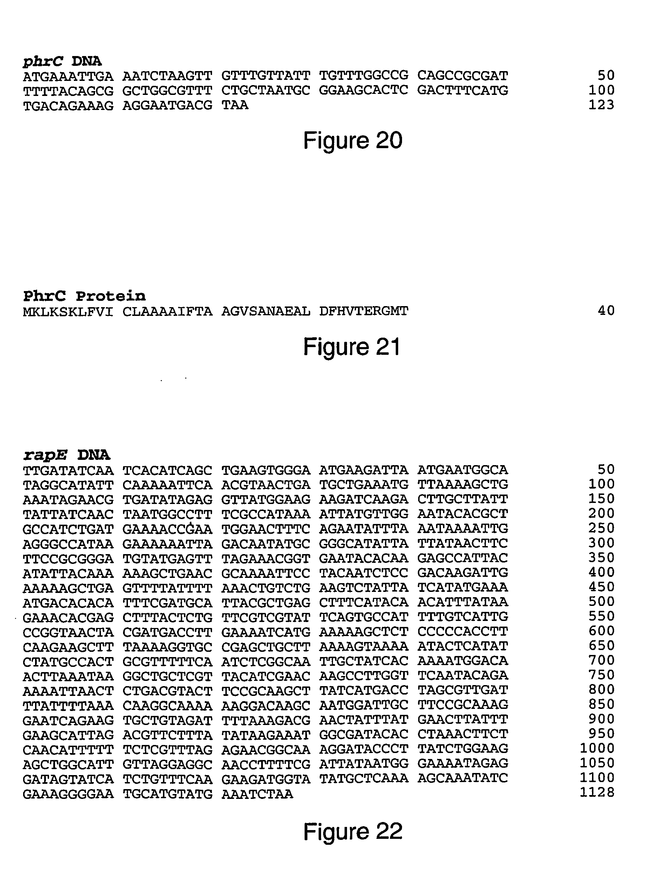

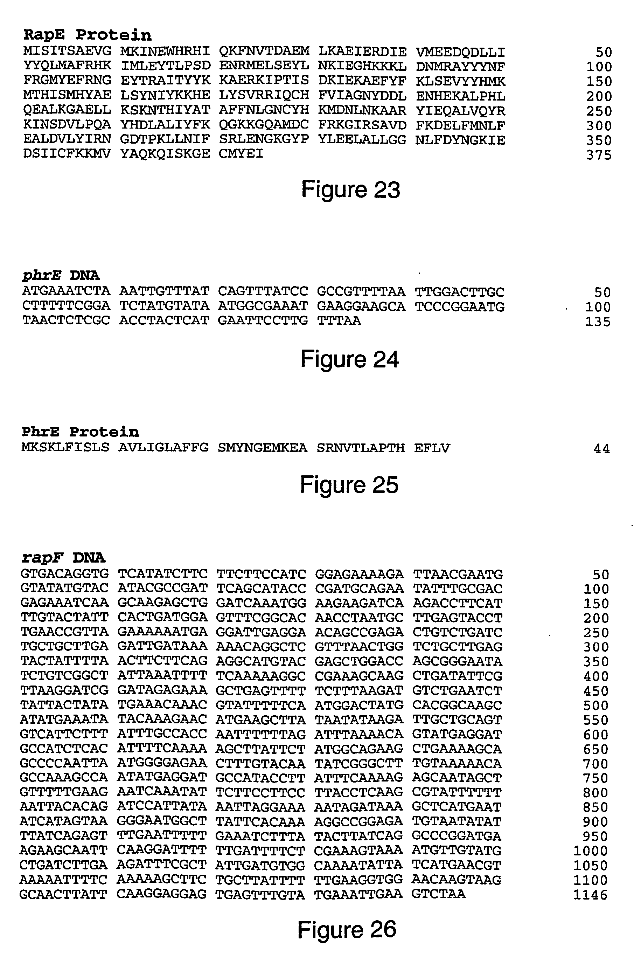

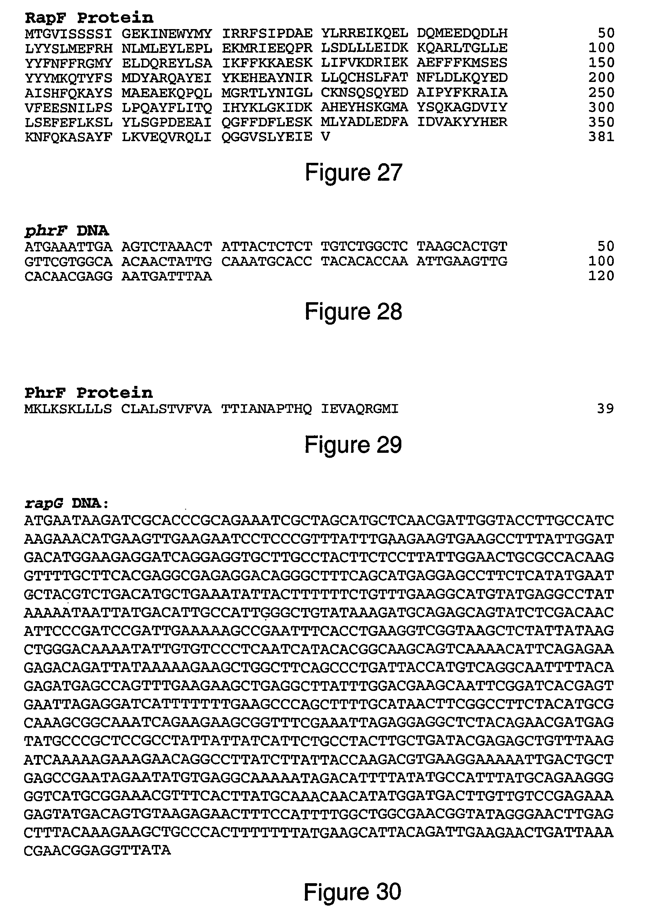

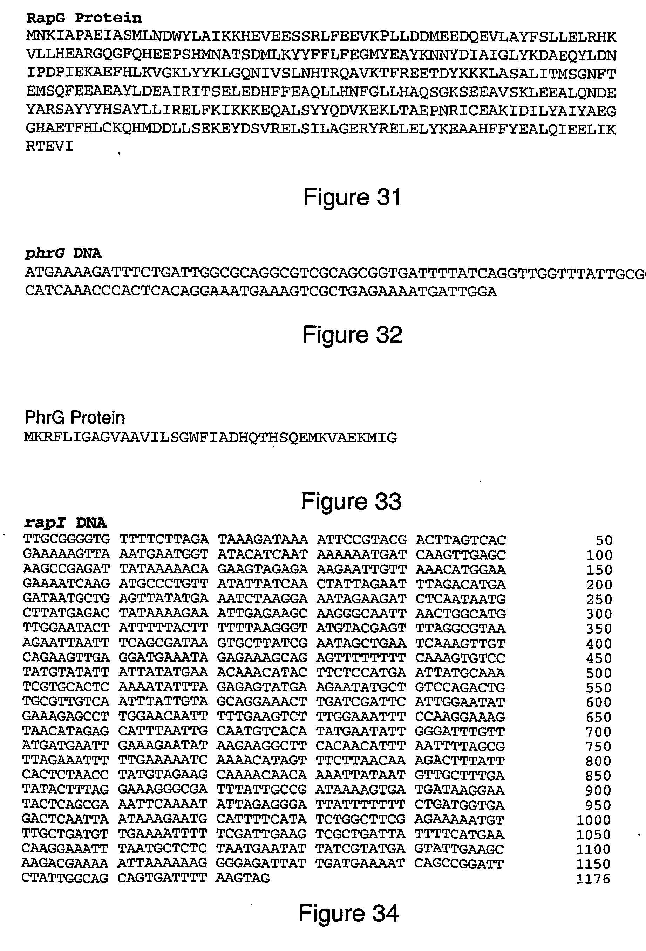

As indicated above, sequences for various Rap and Phr proteins and the genes that encode them are shown in Figures 12 through 37.

### Proteins of Interest

The present invention is particularly useful in enhancing the intracellular and/or extracellular production of proteins. In some embodiments, the protein is homologous (i.e. native or naturally occuring in the host all) while in other embodiments, it is heterologous (ie. it does not naturally occur in the host all). The present invention finds use in the production of various proteins, including but not limited to hormones, enzymes, growth factors, cytokines, antibodies, and the like. The present invention finds particular use in the production of enzymes, including but not limited to hydrolases, such as proteases, esterases, lipases, phenol oxidase, permeases, amylases, pullulanases, cellulases, glucose isomerase, laccases and protein disulfide isomerases. However, the present invention also finds use in the production of hormones, including but are not limited to, follicle-stimulating hormone, luteinizing hormone, corticotropin-releasing factor, somatostatin, gonadotropin hormone, vasopressin, oxytocin, erythropoietin, insulin and the like.

Growth factors are proteins that bind to receptors on the cell surface, with the primary result of activating cellular proliferation and/or differentiation. In addition to enzymes and hormones, the present invention finds use in the production of growth factors including but are not limited to, platelet-derived growth factor, epidermal growth factor, nerve growth factor, fibroblast growth factors, insulin-like growth factors, transforming growth factors, etc. Cytokines are a unique family of growth factors. Secreted primarily from leukocytes, cytokines stimulate both the humoral and cellular immune responses, as well as the activation of phagocytic cells. Cytokines include, but are not limited to, colony stimulating factors, the interleukins (*e.g*., IL-1 [α and β], IL-2 through IL-13) and the interferons (α, β and γ). Human Interleukin-3 (IL-3) is a 15 kDa protein containing 133 amino acid residues. IL-3 is a species-specific colony stimulating factor which stimulates colony formation of megakaryocytes, neutrophils, and macrophages from bone marrow cultures.

In addition to the above proteins, it is contemplated that the present invention will find use in embodiments involving antibodies. Antibodies include, but are not limited to, immunoglobulins from any species from which it is desirable to produce large quantities, It is especially preferred that the antibodies are human antibodies. Immunoglobulins may be from any class (*i*.*e*., IgG, IgA, IgM, IgE, or IgD).

In some embodiments of the present invention, the protein of interest is fused to a signal peptide. Signal peptides from two secretory pathways are specifically contemplated by the present invention. The first pathway is the sec-dependent pathway. This pathway is well characterized and a number of putative signal sequences have been described. It is intended that all sec-dependent signal peptides be encompassed by the present invention. Specific examples include, but are not limited to the AmyL and the AprE sequences. The AmyL sequence refers to the signal sequence for α-amylase and AprE refers to the AprE signal peptide sequence (AprE is subtilisin [also referred to as alkaline protease] of *B. subtilis*). Any signal sequence derived from any source may be used as long as it is functional (*i*.*e*., directs the protein of interest into the secretory pathway), in the host cell.

### Host Cells

The present disclosure provides host microorganisms and expression means for the expression, production and secretion of desired proteins in Gram-positive microorganisms, such as members of the genus *Bacillus.*

In a general embodiment, the present methods find use in enhancing the expression and/or secretion of any protein of interest produced intracellularly or secreted via the Sec-dependent secretion pathway. Thus, any protein of interest that may be fused to a Sec-dependent signal peptide by recombinant DNA methods finds use in the present invention.

In preferred embodiments, the host cell is rendered capable of enhanced expression and/or secretion of a protein of interest by transforming the host cell with nucleotide sequences encoding at least one protein in the Rap-Phr system or modified sequence(s) thereof. In some embodiments, the protein of interest is endogenous, while in other embodiments, it is heterologous. In some embodiments, the protein of interest is a chimeric protein in which a native protein of interest is fused to a Sec-dependent signal sequence. In a preferred embodiment, the at least one Rap-Phr gene is operably linked to a promoter. In some embodiments, the promoter is constitutive, while in other embodiments, it is inducible. A preferred promoter is the *B. subtilis* aprE promoter. It is further contemplated that by varying the level of induction of an inducible promoter it is possible to modulate the expression of the gene product and thereby modulate the secretion of the protein of interest.

In some embodiments, the host cell is a Gram-positive cell. In preferred embodiments, the Gram-positive cell is a member of the genus *Bacillus.* As used herein, the genus *Bacillus* includes all members known to those of skill in the art, including but not limited to *B. subtilis, B. licheniformis, B. lentus, B. brevis, B. stearothermophilus, B. amyloliquefaciens, B. halodurans, B. clausii, B. coagulans, B. circulans, B. megaterum, B. lautus,* and *B. thuringiensis.* In some particularly preferred embodiments, the member of the genus *Bacillus* is selected from the group consisting of *B. subtilis, B. clausii,* and *B. licheniformis.*

### rap Genes and Rap Proteins

In some embodiments of the present invention, at least one *rap* gene or gene product, the gene being selected from rapA, rapB, rapE, rapF, rapG, rapI, and rapK, is "non-functional," in that the gene or gene Product is unable to exert its known function.

In some preferred embodiments, the *rap* gene is modified. As used herein, the terms "modified *rap* sequence" and modified *rap* genes" are used interchangeably and refer to at least one deletion, insertion, interruption and/or mutation of the naturally occurring *rap* nucleic acid sequence. The expression product of the modified sequence may be a truncated protein if the modification is a deletion or interruption of the sequence or mutation altering its functionality. In some preferred embodiments, the modified Rap protein does not retain biological activity. In alternative embodiments, the expression product of the modified sequence is an elongated protein (*e.g*., in embodiments in which the modification is an insertion into the nucleic acid sequence). An insertion may lead to a truncated protein as the expression product if the insertion results in the formation of a stop codon. Thus, as known in the art, depending upon the nature of the insertion, an insertion may result in either a truncated protein or an elongated protein as an expression product.

In alternative embodiments, the *rap* gene product is rendered non-functional. In some embodiments, the *rap* gene product comprises mRNA, while in other embodiments, it comprises a protein product. In some embodiments the mRNA is inactivated by contacting it with an antisense RNA, an RNAse or an RNA binding protein that blocks translation. Thus, in some embodiments, Rap protein is non-functional due to a modification in the rap gene. However, in other embodiments, the non-functionality results from the presence of an antagonist and/or a blocker molecule.

### phr Genes and Phr Gene Product

In some embodiments of the present invention, at least one *phr* gene or gene product is overexpressed. In some embodiments, the *phr* gene is overexpressed by increasing the copy number of the selected *phr* gene. In other embodiments, the phr gene is put under the control of a stronger promoter, while in still further embodiments, it has a stronger ribosome binding site.

In alternative embodiments, the *phr* gene product is added to the growth medium of the host cells. In some embodiments, the *phr* gene products, Phr pentapeptides (shown in Table II) are added. In these embodiments, the Phr protein and/or Phr pentapeptides may be made synthetically or are isolated from a natural source. Thus, the exogenous phr gene products are present in elevated amounts in the culture medium to enhance protein production and/or secretion.

### Transformation of Bacillus Host Cells

In some embodiments of the present invention, nucleic acid encoding at least one polypeptide of interest is introduced into a host cell via an expression vector capable of replicating within the host cell. Suitable replicating plasmids for *Bacillus* known in the art (*See e.g.,* Harwood and Cutting (eds), Molecular Biological Methods for Bacillus, John Wiley & Sons, [1990], in particular, chapter 3; suitable replicating plasmids for *B. subtilis* include those listed on page 92). Although there are technical hurdles, those of skill in the art know that there are several strategies for the direct cloning of DNA in *Bacillus.*

Methods known in the art to transform *Bacillus,* include such methods as plasmid marker rescue transformation, involves the uptake of a donor plasmid by competent cells carrying a partially homologous resident plasmid (Contente et al., Plasmid 2:555-571 [1979]; Haima et al., Mol. Gen. Genet., 223:185-191 [1990]; Weinrauch et al., J. Bacteriol., 154:1077-1087 [1983]; and Weinrauch et al., J. Bacteriol., 169:1205-1211 [1987]). In this method, the incoming donor plasmid recombines with the homologous region of the resident "helper" plasmid in a process that mimics chromosomal transformation.

Another method involving transformation by protoplast transformation is known in the art (*See*, Chang and Cohen, Mol. Gen. Genet., 168:111-115 [1979], for *B. subtilis*; Vorobjeva et al., FEMS Microbiol. Lett., 7:261-263 [1980], for *B. megaterium*; Smith et al., Appl. Env. Microbiol.,51:634 [1986], for *B. amyloliquefaciens*; Fisher et al., Arch. Microbiol., 139:213-217 [1981], for *B. thuringiensis*; McDonald [1984] J. Gen. Microbiol., 130:203 [1984], for *B. sphaericus*; and Bakhiet *et al.,* 49:577 [1985] *B. larvae*). In addition, Mann *et al.,* (Mann et al., Curr. Microbiol., 13:131-135 [1986]) describe transformation of *Bacillus* protoplasts, and Holubova (Holubova, Microbiol., 30:97 [1985]) describe methods for introducing DNA into protoplasts using DNA-containing liposomes. In some preferred embodiments, marker genes are used in order to indicate whether or not the gene of interest is present in the host cell.

In addition to these methods, in other embodiments, host cells are directly transformed. In "direct transformation," an intermediate cell is not used to amplify, or otherwise process, the DNA construct prior to introduction into the host (*i*.*e*., Bacillus) cell. Introduction of the DNA construct into the host cell includes those physical and chemical methods known in the art to introduce DNA into a host cell without insertion into a plasmid or vector. Such methods include but are not limited to calcium chloride precipitation, electroporation, naked DNA, liposomes and the like. In yet other embodiments, the DNA constructs are co-transformed with a plasmid without being inserted into the plasmid.

### Vectors/Plasmids

For expression, production and/or secretion of protein(s) of interest in a cell, an expression vector comprising at least one copy of a nucleic acid encoding the heterologous and/or homologous protein(s), and preferably comprising multiple copies, is transformed into the cell under conditions suitable for expression of the protein(s).

In preferred embodiments of the present invention, expression vectors used to express the Phr proteins in Gram-positive microorganisms comprise at least one promoter associated with a Gram-positive Phr protein, wherein the promoter is functional in the host cell. In one embodiment, the promoter is the wild-type promoter for the selected secretion factor, while in an alternative embodiment, the promoter is heterologous to the secretion factor, but still functional in the host cell.

Additional promoters associated with heterologous nucleic acid encoding desired proteins or polypeptides also find use in the present invention and may be introduced via recombinant DNA techniques known in the art. In one embodiment of the present invention, the host cell is capable of overexpressing a heterologous protein or polypeptide and nucleic acid encoding one or more recombinantly introduced secretion factor(s). In one preferred embodiment of the present invention, nucleic acid encoding a Phr protein or a mutagenized Rap protein is stably integrated into the microorganism genome. In another embodiment, the host cell is engineered to overexpress a Phr protein and nucleic acid encoding the heterologous protein or polypeptide is introduced via recombinant DNA techniques. The present disclosure further encompasses Gram-positive host cells that have one or more Rap proteins inactivated.

In a preferred embodiment, the expression vector contains a multiple cloning site cassette which preferably comprises at least one restriction endonuclease site unique to the vector, to facilitate ease of nucleic acid manipulation. In a preferred embodiment, the vector also comprises one or more selectable markers. In yet another embodiment, a multicopy replicating plasmid is for integration of the plasmid into the Bacilllus genomic DNA using methods known in the art.

### Identification of Transformants

Although the presence/absence of marker gene expression suggests that the gene of interest is also present, its presence and expression should be confirmed. For example, if the nucleic acid encoding a Phr protein is inserted within a marker gene sequence, recombinant cells containing the insert can be identified by the absence of marker gene function. Alternatively, in other embodiments, a marker gene is placed in tandem with nucleic acid encoding the Phr protein under the control of a single promoter. Expression of the marker gene in response to induction or selection usually indicates expression of the Phr protein as well.

Alternatively, host cells which contain the coding sequence for a Phr protein and/or express the protein of interest may be identified by any means known to those of skill in the art. These procedures include, but are not limited to, DNA-DNA or DNA-RNA hybridization and protein bioassay or immunoassay techniques, including, but not limited to membranebased, solution-based, or chip-based technologies for the detection and/or quantification of the nucleic acid or protein.

In other embodiments, the presence of the Phr protein polynucleotide sequence is detected by DNA-DNA or DNA-RNA hybridization or amplification using probes, portions or fragments derived from the polynucleotide encoding any one of the Phr proteins.

The presence of an inactivated or non-functional Rap protein may be detected by methods known in the art. For example, the hyperexpression of RAPs can cause expression of a spo⁻ phenotype, which can be detected utilizing methods known in the art. Alternatively, *in vitro* detection of the dephosphorylation of spoOF∼P to spoOF may indicate the presence of the Rap protein.

### Measuring Gene Products

There are various assays known to those of skill in the art for detecting and measuring activity of intracellularly and extracellularly expressed polypeptides (*e.g*., the protein of interest and/or phr gene products) which find use with the present invention. In particular, for proteases, there are assays based upon the release of acid-soluble peptides from casein or hemoglobin measured as absorbance at 280 nm or colorimetrically using the Folin method (*See e.g*., Bergmeyer et al., Methods of Enzymatic Analysis, vol. 5, [Peptidases, Proteinases and their Inhibitors], Verlag Chemie, Weinheim [1984]). Other assays involve the solubilization of chromogenic substrates (*See*, Ward, in Fogarty (ed), Proteinases, in Microbial Enzymes and Biotechnology Applied Science, London, pp. 251-317 [1983]).

Means for determining the levels of secretion of a heterologous or homologous protein in a host cell and detecting expressed proteins include, but are not limited to immunoassay methods that utilize either polyclonal or monoclonal antibodies specific for the protein. Examples include enzyme-linked immunosorbent assay (ELISA), radioimmunoassay (RIA), fluorescent immunoassays (FA), and fluorescent activated cell sorting (FACS), as well as Western blots, etc. These assay systems are well known in the art (*See e.g*., Maddox et al., J. Exp. Med., 158:1211 [1983]). In some particularly preferred embodiments of the present invention, secretion is greater using the methods and compositions provided herein than when using untransformed host cells or unmodified media.

### Protein Purification

In preferred embodiments, the cells transformed with polynucleotide sequences encoding heterologous or homologous protein or endogenously having said protein are cultured under conditions suitable for the expression and recovery of the encoded protein from the cell culture medium. In some embodiments, other recombinant constructions may join the heterologous or homologous polynucleotide sequences to nucleotide sequence encoding a polypeptide domain which will facilitate purification of soluble protein (*e.g*., tags of various sorts) (Kroll et al., DNA Cell. Biol., 12:441-53 [1993]).

Such purification facilitating domains include, but are not limited to, metal chelating peptides such as histidine-tryptophan modules that allow purification on immobilized metals (Porath, Prot. Expr. Purif., 3:263-281 [1992]), protein A domains that allow purification on immobilized immunoglobulin, and the domain utilized in the FLAGS extension/affinity purification system (Immunex Corp, Seattle WA). The inclusion of a cleavable linker sequence such as Factor XA or enterokinase (Invitrogen, San Diego, CA) between the purification domain and the heterologous protein can be used to facilitate purification.

From the above, it is clear that the present disclosure provides genetically engineered Gram-positive host microorganisms comprising preferably non-revertable mutations, including gene replacement, in at least one gene encoding a *phr* and/or *rap* gene. Compositions useful in enhancing protein production from a host cell are provided herein. In the preferred embodiments, the host cells is a member of the genus *Bacillus.* In some preferred embodiments, the microorganism is also genetically engineered to produce a desired protein or polypeptide. In one such preferred embodiment, the Gram-positive microorganism is a member of the genus *Bacillus.*

The present invention further provides the advantage that it finds use in shifting the proportion of the host cell population to increase the number of producing a protein of interest.

### EXPERIMENTAL

The following examples are provided in order to demonstrate and further illustrate certain preferred embodiments and aspects of the present invention and are not to be construed as limiting the scope thereof.

In the experimental disclosure which follows, the following abbreviations apply: °C (degrees Centigrade); rpm (revolutions per minute); H₂O (water); dH₂O (deionized water); (HCl (hydrochloric acid); aa (amino acid); bp (base pair); kb (kilobase pair); kD (kilodaltons); gm (grams); µg (micrograms); mg (milligrams); ng (nanograms); µl (microliters); ml (milliliters); mm (millimeters); nm (nanometers); µm (micrometer); M (molar); mM (millimolar); µM (micromolar); U (units); V (volts); MW (molecular weight); sec (seconds); min(s) (minute/minutes); hr(s) (hour/hours); MgCl₂ (magnesium chloride); NaCl (sodium chloride); OD₂₈₀ (optical density at 280 nm); OD₆₀₀ (optical density at 600 nm); PAGE (polyacrylamide gel electrophoresis); PBS (phosphate buffered saline [150 mM NaCl, 10 mM sodium phosphate buffer, pH 7:2]); PEG (polyethylene glycol); PCR (polymerase chain reaction); RT-PCR (reverse transcription PCR); SDS (sodium dodecyl sulfate); Tris (tris(hydroxymethyl)aminomethane); w/v (weight to volume); v/v (volume to volume); LA medium (per liter: Difco Tryptone Peptone 20g, Difco Yeast Extract 10g, EM Science NaCl 1g, EM Science Agar 17.5g, dH20 to 1 L); ATCC (American Type Culture Collection, Rockville, MD); Clontech (CLONTECH Laboratories, Palo Alto, CA); Difco (Difco Laboratories, Detroit, MI); GIBCO BRL or Gibco BRL (Life Technologies, Inc., Gaithersburg, MD); Invitrogen (Invitrogen Corp., San Diego, CA); NEB (New England Biolabs, Beverly, MA); Sigma (Sigma Chemical Co., St. Louis, MO); Takara (Takara Bio Inc. Otsu, Japan); Roche Diagnostics (Roche Diagnostics, a division of F. Hoffmann La Roche, Ltd., Basel, Switzerland); EM Science (EM Science, Gibbstown, NJ); Qiagen (Qiagen, Inc., Valencia, CA); and Stratagene (Stratagene Cloning Systems, La Jolla, CA).

In the Examples that follow, the cultures, media, and methods described below were used.

### Plasmids, Bacterial Strains and Media

Table 1 lists the plasmids and bacterial strains used during the development of the present invention. TY medium (tryptone/yeast extract) contained Bacto tryptone (1 %), Bacto yeast extract (0.5%) and NaCl (1%). Extracellular proteolytic activity was tested on TY agar (1.5% agar) with skim milk added (1%). When required, media for *B. subtilis* were supplemented with kanamycin (Km; 5 µg/ml), spectinomycin (Sp; 100 µg/ml), or erythromycin (1 µg/ml).

**Table I. Bacterial Strains**

| **Strains** | **Relevant Properties** |
|---|---|
| *B. subtilis* | |
| 168* | *trpC2* |
| AL | like 168::pAL; *aprE-lacZ; Em^{r}* |
| *degU32* (Hy)** | like 168; *degU32* (Hy); *Km^{r}* |
| *ΔrapE* | like 168::pUCE5-Sp; *rapE-*Sp; *Sp^{r}* |
| | |
| *ΔrapE degU32* (Hy) | like 168::pUCE5-Sp; *rapE-*Sp; *degU32* (Hy); *Km^{r} Sp^{r}* |
| Δ*rapEAL* | like 168::pUCE5-Sp; *rapE-*Sp *aprE-lacZ; Em^{r}; Sp^{r}* |
| *ΔrapE* AL *degU32* (Hy) | like 168::pUCE5-Sp; *rapE-*Sp *aprE-lacZ; Em^{r}; Sp^{r} degU32*(Hy); *Km^{r}* |
| *ΔrapA* | like 168:: pUKA-Cm; *rapA-*Cm; *Cm^{r}* |
| *ΔrapA* AL | like 168::pUKA-Cm; *rapA-*Cm *aprE-lacZ; Em^{r}; Cm^{r}* |
| *ΔrapA* AL *degU32* (Hy) | like 168::pUKA-Cm; *rapA-*Cm *aprE-lacZ; Em^{r}; Cm^{r} degU32* (Hy); *Km^{r}* |
| Δ*rapF* | like 168:: pUKF-Sp; *rapF*-Sp; *Cm^{r}* |
| Δ*rapF* AL | like 168::pUKF-Sp; *rapF*-Sp *aprE-lacZ; Em^{r}; Cm^{r}* |
| Δ*rapF* AL *degU32* (Hy) | like 168::pUKF-Sp; *rapF*-Sp *aprE-lacZ; Em^{r}; Cm^{r} degU32(Hy); Km^{r}* |
| *XphrE* | like 168::pHRE; *amyE::xylA-phrE, Cm^{r}* |
| *XphrE AL* | like 168::pHRE; *amyE::xylA-phrE, Cm^{r}; aprE-lacZ; Em^{r}* |
| *XphrEAL degU32* (Hy) | like 168::pHRE; *amyE::xylA-phrE, Cm^{r}; aprE-lacZ; Em^{r}; degU32* (Hy); *Km^{r}* |
| *XphrF* | like 168::pHRF; *amyE::xylA-phrF, Cm^{r}* |
| *XphrF* AL | like 168::pHRF; *amyE::xyIA-phrF, Cm^{r}; aprE-lacZ; Em^{r}; degU32* (Hy); *Km^{r}* |
| *XphrF AL degU32* (Hy) | like 168::pHRF; *amyE::xylA-phrF, Cm^{r}; aprE-lacZ; Em^{r}* |

| | |
|---|---|
| **See*, Kunst *et al.* (1997), *supra.* ***See*, Antelmann et al., Genome Res., 11:1484-502 (2001). | |

### DNA and RNA Techniques

Procedures for DNA purification, restriction, llgation, agarose gel electrophoresis, and transformation of *E*. *coli* were carried out as known in the art (*See* e.g., Sambrook et al., Molecular Cloning, A Laboratory Manual, Cold Spring Harbor Laboratory Press, Cold Spring Harbor, NY [1989]). Enzymes were from Roche Diagnostics. *B. subtllis* was transformed as known in the art (*See*, Bron and Venema, Mutat. Res., 15:1-10 [1972]). For a high reliability, Pwo polymerase (Roche Diagnostics BmgH) was used. To construct *B. subtfils* Δ*rapE,* a fragment comprising the *rapE* gene was amplified with the primers *rapEb* (5'-CCA TCG ATG GAT CCC ACA TCA GCTGAA GTG GGA ATG-3') and *rapEb* (5'-CGG GAT CCA TCGATG GTT GCC TCC TAA CAA TGC CAG C -3'). The amplified fragment was cleaved with *Bam*HI, and ligated into the corresponding sites of pUC7, resulting in pUCE5. Next, the *rapE* gene of the latter plasmid was interrupted by a spectinomycin resistance marker, resulting in the plasmid pUCE5-Sp. Next, the chromosomal *rapE* gene of *B. subtilis* was disrupted by replacement recombination of pUCE5-Sp with the *rapE* gene of *B. subtilis* 168.

To construct *B. subtilis* Δ*rapA,* a fragment comprising the *rapA* gene was amplified with the primers *rap*A-1 (5'-AGA ATT CAA GCT TAG AAG ACA TCG AAG GG-3') and *phrA*-1 (5'-ATC GGA TCC TTA TGT TTG ATT GCG TGC CGG3'). The amplified fragment was cleaved with *Hind*III and *Bam*HI, and ligated into the *Hind*III and *Bgl*II sites of pUK21, resulting in pUKA. Next, the *rapA* gene of the latter plasmid was interrupted by a chloramphenicol marker, resulting in the plasmid pUKA-Cm. Next, the chromosomal *rapA* gene of *B. subtilis* was disrupted by replacement recombination of pUKA-Cm with the *rapA* gene of *B*. *subtilis* 168.

To construct *B. subtllis* Δ*rapF,* a fragment comprising the *rapF* gene was amplified with the primers *rapF-1* (5'-AGT CTC TAG ATA TGA CAT ATA TAG AAT TAT GGG AGG G-3') and *rapF-2* (5'-ATG CGG ATC CGT CGC ACT CGA CGT TTC GAA CTC CG). The amplified fragment was cleaved with *Xba*I and *Bam*HI, and ligated into the *Xba*I and *Bgl*II sites of pUK21, resulting in pUKF. Next, a 96 bp internal *Hind*III fragment from the *rapF* gene of the latter plasmid was replaced by a spectinomycin marker, resulting in the plasmid pUKF-Sp. Next, the chromosomal *rapF* gene of *B. subtilis* was disrupted by replacement recombination of pUKF-Cm with the *rapF* gene of *B. subtilis* 168.

To construct *B. subtilis XphrE,* a fragment comprising the *phrE* gene was amplified with the primers 5'E-*Xba*I (5'-TAT ATC TAG ATC GAA AGG GGA ATG CAT GTA TGA AAT C-3') and 3'E-*Bam*HI (5'- TTT AGG ATC CAG CGC TTA AAC AAG GAA TTC ATG AGT AGG TGC G-3'). The amplified fragment was cleaved with Xbal and *Bam*HI, and ligated into the *Spe*I and *Bam*HI sites of pX (*See*, Kim et al., Gene 181:71-76 [1996]), resulting In pHRE. Next, the chromosomal *amyE* gene of *B. subtilis* 168 was disrupted by replacement recombination of pHRE, containing a xylose-Inducible *phrE* gene.

To construct *B. subtilis* X*phrF,* a fragment comprising the *phrF* gene was amplified with the primers 5'F-*Xba*I (5'- TTA ATC TAG AAG GAG GAG TGA GTT TGT ATG AAA TTG-3') and 3'F-*Bam*HI (5'- AAT TGG ATC CTT AAG TTA AAT CAT TCC TCG TTG TGC AAC TTC-3'). The amplified fragment was cleaved with Xbal and *Bam*HI, and ligated into the *Spe*I and *Bam*HI sites of pX (*See*, Kim *et al., supra*), resulting In pHRF. Next, the chromosomal *amyE* gene of *B. subtllis* 168 was disrupted by replacement recombination of pHRF, containing a xylose-inducible *phrF* gene.

In some experiments, the *degU32*(Hy) mutation or the transcriptional *aprE-lacZ* fusion, or both, were introduced in *B. subtilis* Δ*rapE,* Δ*rapA, ΔrapF, XphrE* or *XphrF* by transformation with chromosomal DNAs of the *B. subtilis* strains 168 *degU32*(Hy) and/or 168 AL (Table I), using methods known In the art. Constructed strains were selected on plates containing the appropriate antibiotic and checked by PCR analysis for correct integration of plasmids into the chromosome.

### β-Galactosldase Assays

Overnight cultures were diluted to an optical density (OD₆₀₀) of 0.1 in fresh TY medium, supplemented with Phr60 pentapeptide, as appropriate. Samples for OD₆₀₀ readings and β-galactosidase activity determinations were taken at hourly intervals. The assay and the calculation of β-galactosidase activity (expressed as units per OD₆₀₀) of the samples was performed as known In the art (*See*, Miller, Experiments In Molecular Genetics, Cold Spring Harbor Laboratory Press, NY, [1982]).

### Protease Activity Assays

Medium of overnight cultures grown In TY medium was diluted ten-fold in buffer containing 10 mM Tris [pH 6.8]; 100 mM NaCl and 1.4 mg/ml azo dye Impregnated collagen (Sigma), and Incubated for one hour at 37°C. Next, the release of the dye was measured by optical density readings at 520 nM as a quantification of the proteolytic activity.

### EXAMPLE 1

### Chromosomally-Encoded Phr Peptides are Involved in the Regulation of aprE

To analyze whether chromosomally-encoded Rap-Phr systems might be Involved in protease production, *B. subtilis* AL (bearing an *aprE-lacZ* transcriptional fusion), was grown in TY medium supplemented with synthetic PhrA, -C, -E, -F, -G, -H, -I, or -K pentapeptides (Table II). As shown by β-galactosidase assays performed with cells harvested In the post-exponential growth phase (*See*, Figure 1), PhrE and PhrF had a positive effect on *aprE* transcription (±135 % relative to wild-type); PhrG, PhrA, PhrK and PhrI had a weak positive effect on *aprE* transcription (±100-120 % relative to wild-type); whereas PhrC had a clear negative effect on *aprE* transcription (± 60 % relative to wild-type). Finally, the growth rates, and cell densities were not significantly affected by the presence of Phr peptides in the culture medium (data not shown). These results indicate that the temporal transcription of *aprE* is regulated by the combined action of several Rap-Phr systems.

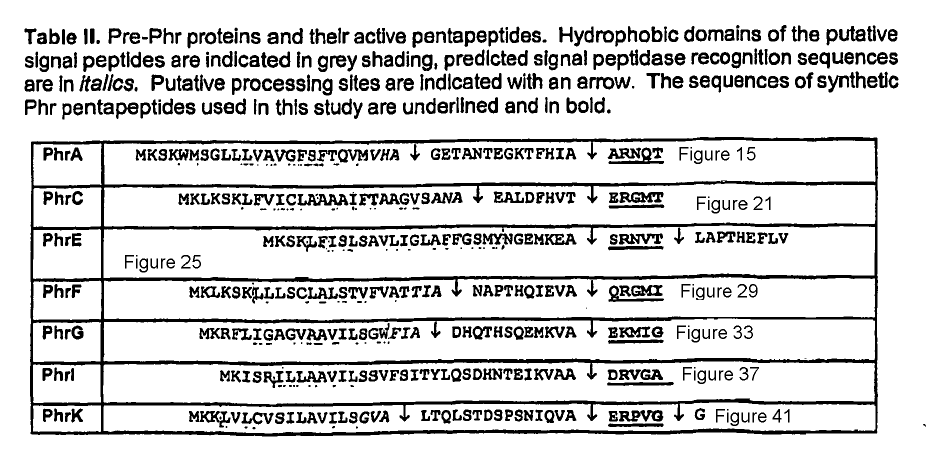

### EXAMPLE 2

### Transcription of aprE is Controlled by the RapE-PhrE System

In this Example, experiments conducted to further assess the effects of Rap-Phr systems on the transcription of *aprE* are described. The RapE-PhrE system was used as a model for two reasons. First, this pair of proteins shows the highest level of homology with the plasmid-encoded Rap60-Phr60 system (data not shown), which was shown during the development of the present invention to be involved in *aprE* transcription. Second, of all Phr pentapeptides tested during the development of the present invention, PhrE was shown to have the strongest positive effect on *aprE* transcription (*See*, Figure 1). To determine the effect of PhrE on *aprE* transcription during growth, synthetic PhrE was added at two concentrations (1 and 10 µg/ml) to the medium of *B. subtilis* AL. The results showed that *aprE* transcription was improved by the addition of PhrE only during the post-exponential growth phase in a PhrE concentration-dependent manner (*See*, Figure 2). Complementary to this result was the observation that *aprE* transcription was increased in a Δ*rapE* genetic background (*See*, Figure 3). It was also noted that in the latter case, *aprE* transcription seemed to be improved during all growth phases. Taken together, these results indicate that RapE is an inhibitor of *aprE* transcription and that RapE itself can be inhibited by the PhrE pentapeptide.

### EXAMPLE 3

### Improved Protease Secretion in a rapE degU32(Hy) Chromosomal Background

A well-known activator of *aprE* transcription is DegU, which is phosphorylated *via* DegS (Msadek et al., J. Bacteriol., 172:824-834 [1990]; and Kunst *et al.,* [1994], *supra*). A *degU32*(Hy) mutation results in stable DegU-P, causing hyper transcription of many protease genes, such as *aprE* (Olmos et al., Mol. Gen. Genet., 253: 562-567 [1997]). The latter mutation, in combination with the *aprE* promoter, finds use in achieving high production yields in strains used for industrial fermentations. To determine whether disruption of *rapE* could improve protease secretion in a strain with a *degU32*(Hy) mutation, a plate assay was used. As shown in Figure 4, Panel A, the halo around colonies of cells containing both mutations was significantly larger than that around colonies of cells containing only the *degU32(Hy)* mutation. Similarly, the proteolytic activity in the medium of overnight cultures of *B. subtilis* 168 Δ*rapE degU32* (Hy) was about 135% of that in *B. subtilis* 168 *degU32* (Hy) (*See*, Figure 4, Panel B). Thus, these results show that protease secretion can be improved by disruption of the *rapE* gene.

### EXAMPLE 4

### Overproduction of Pre-PhrE and Pre-PhrF Can Increase the Transcription of aprE

To analyze whether overproduction of PhrE and PhrF can increase *aprE* transcription, *B. subtilis* AL (bearing an *aprE-lacZ* transcriptional fusion) *B. subtilis XphrE* AL (xylose-inducible production of pre-PhrE; *See,* Figure 5, Panel A) and *B. subtilis XphrF* AL (xylose-inducible production of pre-PhrF; *See,* Figure 5, Panel A), were grown in TY medium supplemented with 1% xylose. As shown by the results of β-galactosidase assays (*See,* Figure 5, Panel B), production of pre-PhrE and pre-PhrF had a positive effect on *aprE* transcription during post-exponential growth (±135-180% relative to wild-type). Similar results were obtained with the same strains carrying in addition the *degU32*(Hy) mutation for hypertranscription of *aprE* (*See,* Figure 5, Panel C). However, the growth rates, and cell densities were not significantly affected by the induction of the *phrE*/*F* genes by xylose (data not shown). These results show that overproduction of pre-PhrE and pre-PhrF can improve *aprE* transcription, most likely by inhibiting RapA and RapF, respectively. However, it is not intended that the present invention be limited to any specific mechanism.

### EXAMPLE 5

### Transcription of aprE is Controlled by RapA

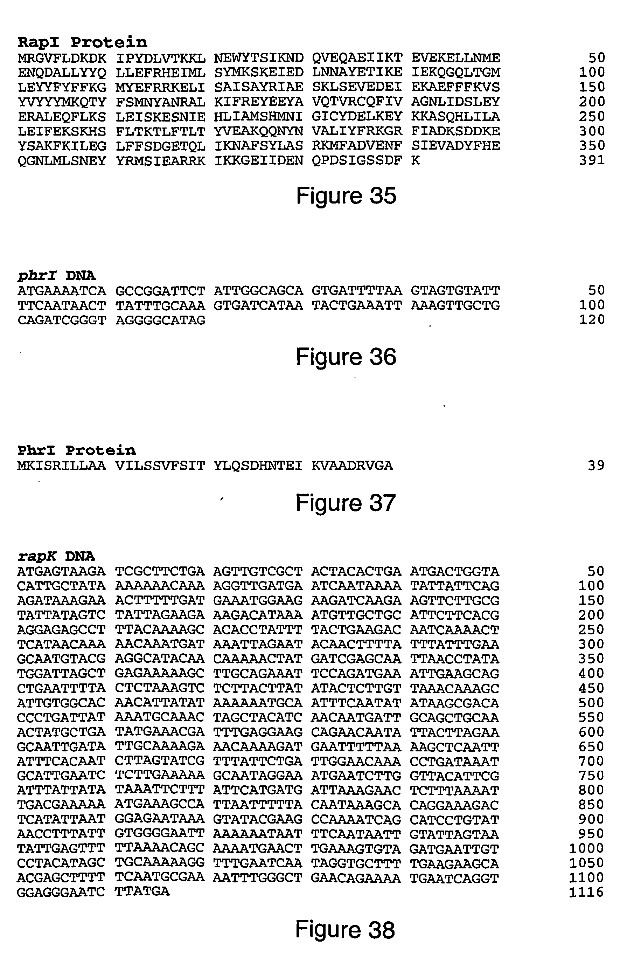

To analyze the effects of RapA on the transcription of *aprE,* the *rapA* gene of the strain *B. subtilis* AL (bearing an *aprE-lacZ* transcriptional fusion) was disrupted by a chloramphenicol marker (*See*, Figure 6, Panel A). Next, *B. subtilis* AL and *B. subtilis* Δ*rapA* AL were grown at 37°C in TY medium. As shown by β-galactosidase assays (*See*, Figure 6, Panel B), disruption of *rapA* had a positive effect on *aprE* transcription during post-exponential growth (±120-200% relative to wild-type). Similar results were obtained with the same strains carrying in addition the *degU32*(Hy) mutation for hypertranscription of *aprE* (*See*, Figure 36, Panel C). However, the growth rates, and cell densities were not significantly affected by the *rapA* disruption (data not shown). Taken together, these results indicate that RapA is an inhibitor of *aprE* transcription, most likely by the dephosphorylation of SpoOF~P, similar to the function of RapE (*See* Figure 8, as well as Perego, [1998], *supra*; and Tzeng *et al.,* [1998], *supra*).

### EXAMPLE 6

### Transcription of aprE is Controlled by RapF

To analyze the effects of RapF on the transcription of *aprE,* the *rapF* gene of the strain *B. subtilis* AL (bearing an *aprE-lacZ* transcriptional fusion) was disrupted by a spectinomycin marker (*See*, Figure 7, Panel A). Next, *B. subtilis* AL and *B. subtilis* Δ*rapF* AL were grown at 37°C in TY medium. As shown by β-galactosidase assay results (Figure 7, Panel B), disruption of *rapF* had a positive effect on *aprE* transcription during post-exponential growth (±110-150% relative to wild-type). Similar results were obtained with the same strains carrying in addition the *degU32*(Hy) mutation for hypertranscription of *aprE* (*See*, Figure 7, Panel C). However, the growth rates, and cell densities were not significantly affected by the *rapF* disruption (data not shown). Taken together, these results indicate that RapF is an inhibitor of *aprE* transcription, but the target of RapF is currently unknown. Indeed, it is not intended that the present invention be limited to any particular mechanism(s).

Various modifications and variations of the described method and system of the invention will be apparent to those skilled in the art. Although the invention has been described in connection with specific preferred embodiments, it should be understood that the invention as should not be unduly limited to such specific embodiments. Indeed, various modifications of the described modes for carrying out the invention that are obvious to those skilled in the art and/or related fields are intended to be within the scope of the present invention.

## Claims

1. A method of altering the amount of a polypeptide of interest expressed from a cell comprising overexpression of one or more *phr* genes selected from the group consisting of *phrA, phrE, phrF, phrG, phrl,* and *phrK* or contacting a host cell with a composition comprising one or more of the Phr proteins selected from the group consisting of PhrA, PhrE, PhrF, PhrG, PhrI, and PhrK.

2. A method of altering the amount of a polypeptide of interest expressed from a cell comprising deletion or inactivation of one or more *rap* genes selected from the group consisting of *rapA, rapE, rapF, rapG, rapI,* and *rapK.*

3. The method of Claim 1 or Claim 2, wherein said polypeptide of interest is native or naturally occurring in the host cell.

4. The method of Claim 3, wherein said native or naturally occurring polypeptide is a protease.

5. The method of Claim 4, wherein said native or naturally occurring polypeptide is AprE.

6. The method of Claim 1 or Claim 2, wherein said polypeptide of interest is a heterologous polypeptide that does not naturally occur in the host cell.

7. The method of Claim 6, wherein said heterologous polypeptide is selected from the group consisting of hydrolases, isomerases, transferases, kinases, phosphatases, hormones, growth factors, cytokines, and antibodies.

8. The method of Claim 7, wherein:
(a) said hydrolase is selected from the group consisting of proteases, cellulases, amylases, and lipases;
(b) isomerase is selected from the group consisting of racemases, epimerases, tautomerases, and mutases.

9. A method for producing a protein of interest comprising the steps of:
(a) culturing under suitable conditions a *Bacillus* host cell comprising:
i) a DNA encoding the protein of interest, wherein said host cell is
ii) transformed with a DNA encoding a protein having the amino acid sequence selected from the group consisting of the sequences as shown in Figures 15, 25, 29, 37, 41 and 33; or a protein having an amino acid sequence at least 85% identical to a protein having the amino acid sequence selected from the group consisting of the sequences as shown in Figures 15, 25, 29, 37, 41 and 33, wherein said protein has a Phr function; and
(b) allowing expression of the protein of interest.

10. A method for producing a protein of interest comprising the steps of:
(a) culturing under suitable conditions a *Bacillus* host cell comprising: a DNA encoding the protein of interest, wherein said host cell lacks a functional *rap* gene or gene product; where in said *rap* gene is selected from the group consisting of *rapA, rapB, rapE, rapF, rapG, rapI,* and *rapK*; and
(b) allowing expression of the protein of interest.

11. A method for production of a protein of interest in a host cell, comprising:
(a) transforming said host cells with a first nucleotide sequence encoding at least one *phr* gene selected from the group consisting of *phrA, phrE, phrF, phrG, phrI*, and *phrK*;
(b) transforming said host cells with a second nucleotide sequence encoding a protein of interest that can be expressed by the host cell; and
(c) culturing said host cells under conditions to express said first and second DNA sequences.

12. The method of Claim 11, further comprising the step of recovering said polypeptide from said culture medium.

13. A method for production of a protein of interest in a host cell, comprising culturing bacterial cells that have been transformed with a recombinant expression vector comprising a first DNA sequence encoding a polypeptide that can be expressed by the bacteria and a second DNA sequence encoding at least one *phr* gene selected from the group consisting of *phrA, phrE, phrF, phrG, phrI*, and *phrK* under conditions to express said first and second DNA sequences.

14. The method of Claim 13, further comprising the step of recovering said protein from said culture medium.

15. A method for production of a protein of interest in a host cell, comprising:
(a) transforming a host cell with a DNA encoding a polypeptide of interest;
(b) contacting said host cell with a Phr pentapeptide selected from the group consisting of PhrA, PhrE, PhrF, PhrG, PhrI, and PhrK; and
(c) culturing said host cells under conditions to express said polypeptide of interest.

## Patentansprüche

1. Verfahren zur Veränderung der Menge eines von einer Zelle exprimierten Polypeptids von Interesse, umfassend die Überexpression von einem oder mehreren aus der aus phrA, phrE, phrF, phrG, phrI und phrK bestehenden Gruppe ausgewählten phr-Genen oder das Kontaktieren einer Wirtszelle mit einer Zusammensetzung, die ein oder mehrere der aus der aus PhrA, PhrE, PhrF, PhrG, Phrl und PhrK bestehenden Gruppe ausgewählten Phr-Proteine umfasst.

2. Verfahren zur Veränderung der Menge eines von einer Zelle exprimierten Polypeptids von Interesse, umfassend die Deletion oder Deaktivierung eines oder mehrerer aus der aus rapA, rapE, rapF, rapG, rapI und rapK bestehenden Gruppe ausgewählter rap-Gene.

3. Verfahren nach Anspruch 1 oder Anspruch 2, worin das Polypeptid von Interesse in der Wirtszelle nativ ist oder natürlich vorkommt.

4. Verfahren nach Anspruch 3, worin das native oder natürlich vorkommende Polypeptid eine Protease ist.

5. Verfahren nach Anspruch 4, worin das native oder natürlich vorkommende Polypeptid AprE ist.

6. Verfahren nach Anspruch 1 oder Anspruch 2, worin das Polypeptid von Interesse ein heterologes Polypeptid ist, das nicht natürlich in der Wirtszelle vorkommt.

7. Verfahren nach Anspruch 6, worin das heterologe Polypeptid aus der aus Hydrolasen, Isomerasen, Transferasen, Kinasen, Phosphatasen, Hormonen, Wachstumsfaktoren, Zytokinen und Antikörpern bestehenden Gruppe ausgewählt ist.

8. Verfahren nach Anspruch 7, worin:
(a) die Hydrolase aus der aus Proteasen, Cellulasen, Amylasen und Lipasen bestehenden Gruppe ausgewählt ist;
(b) die Isomerase aus der aus Racemasen, Epimerasen, Tautomerasen und Mutasen bestehenden Gruppe ausgewählt ist.

9. Verfahren zur Herstellung eines Proteins von Interesse, umfassend die folgenden Schritte:
(a) das Kultivieren einer Folgendes umfassenden Bacillus-Wirtszelle unter geeigneten Bedingungen:
(i) eine für das Protein von Interesse kodierende DNA, worin die Wirtszelle
(ii) mit einer DNA transformiert ist, die für ein Protein mit der Aminosäuresequenz, die aus der aus den Sequenzen, die in den Figuren 15, 25, 29, 37, 41 und 33 gezeigt werden, bestehenden Gruppe ausgewählt ist, oder für ein Protein mit einer Aminosäuresequenz, die zu zumindest 85 % mit einem Protein mit der Aminosäuresequenz, die aus der aus den Sequenzen, die in den Figuren 15, 25, 29, 37, 41 und 33 gezeigt werden, bestehenden Gruppe ausgewählt ist, identisch ist, kodiert, worin das Protein eine Phr-Funktion aufweist; und
(b) das Zulassen der Expression des Proteins von Interesse.

10. Verfahren zur Herstellung eines Proteins von Interesse, umfassend die folgenden Schritte:
(a) das Kultivieren einer Bacillus-Wirtszelle, die eine DNA umfasst, die für das Protein von Interesse kodiert, unter geeigneten Bedingungen, worin der Wirtszelle ein funktionelles rap-Gen oder -Genprodukt fehlt; worin das rap-Gen aus der aus rapA, rapB, rapE, rapF, rapG, rapI und rapK bestehenden Gruppe ausgewählt ist; und
(b) das Zulassen der Expression des Proteins von Interesse.

11. Verfahren zur Herstellung eines Proteins von Interesse in einer Wirtszelle, umfassend:
(a) das Transformieren der Wirtszellen mit einer ersten Nucleotidsequenz, die für zumindest ein aus der aus phrA, phrE, phrF, phrG, phrI und phrK bestehenden Gruppe ausgewähltes phr-Gen kodiert;
(b) das Transformieren der Wirtszellen mit einer zweiten Nucleotidsequenz, die für ein Protein von Interesse kodiert, das durch die Wirtszelle exprimiert werden kann; und
(c) das Kultivieren der Wirtszellen unter Bedingungen, die zur Expression der ersten und der zweiten DNA-Sequenz führen.

12. Verfahren nach Anspruch 11, weiters umfassend den Schritt des Gewinnens des Polypeptids aus dem Kulturmedium.

13. Verfahren zur Herstellung eines Proteins von Interesse in einer Wirtszelle, umfassend das Kultivieren von Bakterienzellen, die mit einem rekombinanten Expressionsvektor transformiert wurden, der eine erste DNA-Sequenz, die für ein Polypeptid kodiert, das von den Bakterien exprimiert werden kann, und eine zweite DNA-Sequenz, die für zumindest ein aus der aus phrA, phrE, phrF, phrG, phrI und phrK bestehenden Gruppe ausgewähltes phr-Gen kodiert, umfasst, unter Bedingungen, die zur Expression der ersten und der zweiten DNA-Sequenz führen.

14. Verfahren nach Anspruch 13, weiters umfassend den Schritt des Gewinnens des Proteins aus dem Nährmedium.

15. Verfahren zur Herstellung eines Proteins von Interesse in einer Wirtszelle, umfassend:
(a) das Transformieren einer Wirtszelle mit einer DNA, die für ein Polypeptid von Interesse kodiert;
(b) das Kontaktieren der Wirtszelle mit einem aus der aus PhrA, PhrE, PhrF, PhrG, Phrl und PhrK bestehenden Gruppe ausgewählten Phr-Pentapeptid; und
(c) das Kultivieren der Wirtszellen unter Bedingungen, die zur Expression des Polypeptids von Interesse führen.

## Revendications

1. Méthode pour modifier la quantité d'un polypeptide d'intérêt exprimé par une cellule comprenant la surexpression d'un ou de plusieurs gènes *phr* sélectionnés dans le groupe consistant en *phrA, phrE, phrF, phrG, phrI* et *phrK* ou bien mettre en contact une cellule hôte avec une composition comprenant une ou plusieurs des protéines Phr sélectionnées dans le groupe consistant en PhrA, PhrE, PhrF, PhrG, PhrI et PhrK.

2. Méthode pour modifier la quantité d'un polypeptide d'intérêt exprimé par une cellule comprenant la délétion ou l'inactivation d'un ou de plusieurs gènes rap sélectionnés dans le groupe consistant en *rapA, rapE, rapF, rapG, rapI* et *rapK*.

3. Méthode selon la revendication 1 ou la revendication 2, dans laquelle ledit polypeptide d'intérêt est natif ou se produit naturellement dans la cellule hôte.

4. Méthode selon la revendication 3, où ledit polypeptide natif ou se produisant naturellement est une protéase.

5. Méthode selon la revendication 4, où ledit polypeptide natif ou se produisant naturellement est AprE.

6. Méthode selon la revendication 1 ou la revendication 2, où ledit polypeptide d'intérêt est un polypeptide hétérologue qui ne se produit pas naturellement dans la cellule hôte.

7. Méthode selon la revendication 6, où ledit polypeptide hétérologue est sélectionné dans le groupe consistant en hydrolases, isomérases, transférases, kinases, phosphatases, hormones, facteurs de croissance, cytokines et anticorps.

8. Méthode selon la revendication 7, où:
(a) ladite hydrolase est sélectionnée dans le groupe consistant en protéases, cellulases, amylases et lipases;
(b) l'isomérase est sélectionnée dans le groupe consistant en racémases, épimérases, tautomérases et mutases.

9. Méthode de production d'une protéine d'intérêt comprenant les étapes de
(a) cultiver sous des conditions appropriées une cellule hôte de Bacillus comprenant:
i) un ADN codant pour la protéine d'intérêt, où ladite cellule hôte est
ii) transformée par un ADN codant pour une protéine ayant la séquence d'acides aminés sélectionnée dans le groupe consistant en les séquences comme indiqué sur les Figures 15, 25, 29, 37, 41 et 33; ou une protéine ayant une séquence d'acides aminés au moins à 85% identique à une protéine ayant la séquence d'acides aminés sélectionnée dans le groupe consistant en les séquences comme indiqué sur les Figues 15, 25, 29, 37, 41 et 33, où ladite protéine a une fonction Phr; et
(b) permettre l'expression de la protéine d'intérêt.

10. Méthode de production d'une protéine d'intérêt comprenant les étapes de:
(a) cultiver sous des conditions appropriées une cellule hôte de Bacillus comprenant: un ADN codant pour la protéine d'intérêt, où manque à la cellule hôte un gène *rap* fonctionnel ou un produit de gène; où ledit gène *rap* est sélectionné dans le groupe consistant en *rapA, rapB, rapE, rapF, rapG, rapI* et *rapK*; et
(b) permettre l'expression de la protéine d'intérêt.

11. Méthode de production d'une protéine d'intérêt dans une cellule hôte, comprenant:
(a) transformer lesdites cellules hôtes avec une première séquence de nucléotides codant pour au moins un gène phr sélectionné dans le groupe consistant en *phrA, phrE, phrF, phrG, phrI* et *phrK*;
(b) transformer lesdites cellules hôtes avec une deuxième séquence de nucléotides codant pour une protéine d'intérêt qui peut être exprimée par la cellule hôte; et
(c) cultiver lesdites cellules hôtes sous des conditions pour exprimer lesdites première et deuxième séquences d'ADN.

12. Méthode selon la revendication 11, comprenant en outre l'étape consistant à récupérer ledit polypeptide dudit milieu de culture.

13. Méthode de production d'une protéine d'intérêt dans une cellule hôte, comprenant la culture de cellules bactériennes qui ont été transformées avec un vecteur d'expression recombinant comprenant une première séquence d'ADN codant pour un polypeptide qui peut être exprimé par les bactéries et une deuxième séquence d'ADN codant pour au moins un gène phr sélectionné dans le groupe consistant en *phrA, phrE, phrF, phrG, phrI* et *phrK* sous des conditions pour exprimer lesdites première et deuxième séquences d'ADN.

14. Méthode selon la revendication 13, comprenant en outre l'étape consistant à récupérer ladite protéine dudit milieu de culture.

15. Méthode de production d'une protéine d'intérêt dans une cellule hôte, comprenant:
(a) transformer une cellule hôte avec un ADN codant pour un polypeptide d'intérêt;
(b) mettre en contact ladite cellule hôte avec un pentapeptide Phr sélectionné dans le groupe consistant en PhrA, PhrE, PhrF, PhrG, PhrI et PhrK; et
(c) cultiver lesdites cellules hôtes sous des conditions pour exprimer ledit polypeptide d'intérêt.
